(19) Europäisches Patentamt
European Patent Office
Office européen des brevets

(11) EP 1 117 419 B1

(12) **EUROPEAN PATENT SPECIFICATION**

(45) Date of publication and mention of the grant of the patent:
**20.01.2010 Bulletin 2010/03**

(21) Application number: **99956502.1**

(22) Date of filing: **29.09.1999**

(51) Int Cl.:
*C12N 9/64* (2006.01) *A61K 38/17* (2006.01)
*C07K 14/46* (2006.01) *A61P 35/00* (2006.01)
*A61P 7/02* (2006.01) *A61P 17/02* (2006.01)

(86) International application number:
**PCT/US1999/022608**

(87) International publication number:
**WO 2000/018421 (06.04.2000 Gazette 2000/14)**

(54) **CONTORTROSTATIN (CN) AND METHODS FOR ITS USE IN PREVENTING METASTASIS AND OTHER CONDITIONS**

CONTORTROSTATIN(CN) SOWIE VERFAHREN ZU SEINER VERWENDUNG IN DER PRÄVENTION VON METASTASEN UND ANDERER ZUSTÄNDE

CONTORTROSTATINE (CN) ET PROCEDES RELATIFS A SON UTILISATION POUR PREVENIR LA METASTASE ET D'AUTRES AFFECTIONS

(84) Designated Contracting States:
**AT BE CH CY DE DK ES FI FR GB GR IE IT LI LU MC NL PT SE**

(30) Priority: **29.09.1998 US 163047**

(43) Date of publication of application:
**25.07.2001 Bulletin 2001/30**

(60) Divisional application:
**09008633.1**

(73) Proprietor: **The University Of Southern California Los Angeles, CA 90007 (US)**

(72) Inventors:
- **MARKLAND, Francis, S., Jr.
Manhattan Beach, CA 90266 (US)**
- **ZHOU, Qing
Alhambra, CA 91801 (US)**

(74) Representative: **Ward, David Ian et al
Marks & Clerk LLP
Alpha Tower
Suffolk Street
Queensway
Birmingham
B1 1TT (GB)**

(56) References cited:
**US-A- 5 731 288**

- **ZHOU Q. ET AL.: "Contortrostatin, a snake venom protein which is an inhibitor of breast cancer progression." MOLEC. BIOLOGY OF THE CELL, vol. 7, no. suppl., 1996, XP009007503**
- **MERCER B. ET AL.: "Contortrostatin, a homodimeric snake venom disintegrin, is a potent inhibitor of osteoclast attachment." J. BONE MIN. RES., vol. 13, no. 3, March 1998 (1998-03), pages 409-414, XP009007481**
- **COUSINS G. ET AL.: "Contortrostatin prevents reocclusion after thrombolytic therapy in a canine model of carotid artery thrombosis." FASEB J., vol. 9, no. 4, 1995, XP009007492**
- **ZHOU Q. ET AL.: "Contortrostatin, a snake venom disintegrin, is an inhibitor of Kaposi's sarcoma progression." PROC. NAT. AMERICAN ASSOC. FOR CANCER RES. MEEETING, vol. 39, 28 March 1998 (1998-03-28) - 1 April 1998 (1998-04-01), XP001146421**
- **DATABASE CAPLUS, 1994:572676 CLARK ET AL.: 'Structurally Distinct Disintegrins Contortrostatin and Multisquamatin Differentially Regulate Platelet Tyrosine Phosphorylation' & J. BIOL. CHEM., vol. 269, no. 35, 1994, pages 21940 - 21943**
- **DATABASE CAPLUS, P90457 CHIRON CORP.: 'DNA Encoding Snake Venom Fibrolase - for use as Thrombolytic Agent' & EP 323 722 A 12 July 1989**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE CAPLUS R06494 BIOGEN INC.: 'Pure Platelet Activation Inhibiting Polypeptide from Snake Venom - used for Preventing Agglutination and release In Vivo or Vitro and new Recombinant DNA Encoding it' & WO 90 08772 A 09 August 1990
- DATABASE CAPLUS, W14083 MOGAM BIOTECHNOLOGY RES. INST.: 'peptide Derived from Korean Salmosa Viper Venom - Useful as Blood Platelet Aggregation Inhibitor, for the Management of Thrombosis' & FR 2 736 266 A1 10 January 1997

## Description

### FIELD OF THE INTENTION

The present invention relates generally to the field of biochemistry and medicine and in particular to the cloning, sequencing, and production of contortrostatin and its precursor.

### BACKGROUND OF THE INVENTION

Breast cancer is one of the leading causes of death among non-smoking women and the spread of the disease from the breast to distant sites is a major cause of death in breast cancer patients. At the time of diagnosis over 60% of breast cancer patients will have disease that has spread from the primary site in the breast to some distant site. Spread of cancer to remote sites, e.g. bone, lungs, liver, brain, a process called metastasis, is a characteristic of malignancy and often leads to inoperable disease. Metastasis is the most common factor leading to death from breast cancer. Control of metastasis offers an important avenue for breast cancer treatment. Cancer cells metastasize through the blood or lymph vessels. The first step of metastasis involves the attachment of cancer cells to tissues around the primary site, i.e., to the extracellular matrix (ECM) via cell surface integrins and other adhesion receptors. Integrins mediate cell-cell and cell-substratum interactions and are involved in bidirectional signaling that links the ECM with cytoskeletal proteins. Integrins play an important role in the interaction of mammary carcinoma cells with the ECM. In the second step, cancer cells secrete digestive enzymes that degrade the surrounding tissues allowing the tumor cells to invade these tissues. Eventually, the tumor cells enter the blood or lymphatic system where they repeat the adhesion and invasion steps at a distant (metastatic) site. At this remote site, tumor cells induce the formation of new blood vessels (a process called neovascularization), in and around the growing tumor. These new blood vessels supply nutrients to the metastatic tumor and allow it to grow. Treatments that block any of these steps should act to inhibit metastasis.

Integrins on cancer cells play important roles in tumor invasion and spread. They are a family of proteins found on the cell surface of many cell types that mediate interactions between cells, and between cells and their surroundings. CN binds to a specific integrin on the surface of blood platelets, and blocks the ability of platelets to adhere to one another (a process called platelet aggregation). Platelets are small fragments of bone marrow cells that are found in the blood stream. They have both beneficial and harmful activities. Their useful action is to stop bleeding following injury by facilitating the formation of a blood clot. But, under certain conditions they are involved in blocking arteries that supply nourishment to the heart - an action that can lead to a heart attack. Integrins are heterodimers, composed of a and β subunits involved in cell-cell and cell-substratum interactions. Integrins serve as receptors for extracellular matrix proteins such as fibronectin, fibrinogen, vitronectin, collagen and laminen. Some of these interactions have been shown to be mediated via an Arg-Gly-Asp (RGD) sequence present in the matrix proteins. Both the a and β subunits are required for fibrinogen binding. For example, one of the members of the superfamily of integrin cell surface receptors is the platelet membrane glycoprotein (GP)IIb/IIIa which interacts with plasma fibrinogen in platelet aggregation. Integrin cell surface receptors have been investigated in the role of platelets in mediating coronary artery thrombosis and rethrombosis in the genesis of acute myocardial infarction [Zucker, M.B., Sci. American 242:86 (1990)]. For platelet aggregation an RGD sequence present in fibrinogen is essential for the interaction with (GP)IIb/IIIa [Ginsberg, M.H. et al., Thrombos. Haemostas. 59:1 (1988)]. Because of its inhibition of platelet aggregation, snake venom has been the subject of various investigations.

A number of proteins purified from venom of snakes of the *Crotalidae* and *Viperidae* families have been found to inhibit glycoprotein (GP)IIb/IIIa mediated platelet aggregation [*see, e.g.,* Huang, T.F. et al., J. Biol. Chem. 262:16157 (1987); Gan, Z.R. et al., J. Biol Chem. 263:19827 (1988); Yasuda, T. et al., J. Am. Coll. Cardiol. 16:714 (1990); Trikha, M. et al., Fibrinolysis 4 (Suppl. 1):105 (1990); Trikha, M. et al., Blood 76 (SuppL 1):479a (1990); Holahan, M.A. et al., Pharmacology 42:340 (1991); Shebuski, R.J. et al., Circulation 82:169 (1990); Yasuda, T. et al., Circulation 83:1038 (1991)]. These proteins, classified as disintegrins, are typically disulfide rich. Moreover, all disintegrins isolated thus with the exception of barbourin [Scarborough, R.M. et al., J. Biol Chem. 266:9359 (1991)] contain an RGD (Arg-Gly-Asp) sequence that has been implicated as being involved in the inhibition of integrin-mediated interactions. In particular, the RGD sequence of the disintegrins may compete for fibrinogen binding sites on the platelet membrane, thereby inhibiting platelet aggregation induced by ADP or other agents.

Nonetheless, there appears to be increasing evidence that disintegrins may have unique surface geometry which facilitates interactions with integrins by mechanisms other than those based solely upon the RGD site. For example, the finding that a mutated, chemically synthesized derivative of echistatin (in which alanine was substituted for arginine in the RGD sequence) still possessed some biological activity, suggests that other regions in the protein may be involved in binding and that there may be some flexibility in the RGD binding site [Connolly, T.M. et al., Circulation 82. (Suppl. III):660 (1990)]. Synthetic RGD peptides, due to their small size, generally do not possess the molecular topography of the disintegrins and therefore cannot interact via the multiplicity of mechanisms likely to be involved in disintegrin binding.

In other investigations, prevention ofreocclusion following thrombolysis using tissue-type plasminogen activator in a canine model system has been reported using 30 μg/kg plus 3 μg/kg/min bitistatin, an 83 amino acid disintegrin derived from the venom *of Bitis arietans* [Shebuski et al., *supra],* or 15 μg/kg/min i.v. echistatin, a 49 amino acid disintegrin derived from the venom of *Echis carinatus* [Holahan et al., *supra].* In the reported methods, an initial bolus of heparin (100 U/kg i.v.) and subsequent hourly boluses of 50 U/kg were used to increase activated partial thromboplastin times at least 1.5-fold over the control. Whereas it had previously been observed that heparin in combination with tissue-type plasminogen activator (tPA) did not affect the incidence of acute reocclusion in this model system, the addition of echistatin or bitistatin lead to dramatic reductions in the incidence of acute thrombotic reocclusion. The administration of heparin was, however, apparently necessary for prevention of acute thrombotic reocclusion.

Similarly, kistrin (a 68 amino acid disintegrin derived from the venom of *Agkistrodon rhodostoma*) was evaluated in conjunction with recombinant tissue-type plasminogen activator in a canine model of coronary artery thrombosis with superimposed high grade stenosis [Yasuda et al. (1991), *supra*]. An effective dose of 4 μg/kg/min was determined to be sufficient to prevent reocclusion. Simultaneous systemic therapeutic heparin anticoagulation was used; the dose of heparin was selected to maintain the activated partial thromboplastin time more than two-fold throughout the experimental observation period.

U.S. Patent 5,066,592 to Huang et al. describes the use of trigramin, a 72 amino acid disintegrin isolated from the venom of *Trimeresurus gramineus,* to inhibit fibrinogen binding to human platelets and thereby to inhibit fibrinogen-induced aggregation of human platelets. Trigramin is also reported to inhibit binding of von Willebrand factor to platelets. Trigramin is reported to inhibit $^{125}$I-fibrinogen binding to ADP (10 uμmolar)-stimulated platelets in a concentration-dependent manner with an $IC_{50}$ of 2.8-5.6 x $10^{-8}$M.

Isolation of an anti-platelet factor applaggin from the venom *of Agkistrodon piscivorus piscivorus* has also been reported [Chao, B.H. et al., Proc. Natl. Acad Sci. USA 86:8050 (1989); Savage, B. et al., J. Biol Chem. 265:11766 (1990)]. Applaggin, unlike trigramin, is reported to inhibit dense-granule secretion in concert with inhibition of platelet aggregation in a dose-dependent manner. While initially described as a homodimer with at least two interchain disulfide bridges [Chao et al. (1989), *supra],* a subsequent report indicated that analysis of purified applaggin by mass spectroscopy showed the presence of applaggin monomers with a mass of 7,666 Daltons and no evidence of dimerization [Wencel-Drake, J.D. et al., Blood 81:62 (1993)].

One disintegrin of particular interest is CN, which has been isolated from the venom of *Agkistrodon contortrix contortrix* (the southern copperhead snake). The originally-reported purification procedure included molecular sieve chromatography on Sephadex G-100 SF, desalting on Sephadex G-25F and reverse phase HPLC. ADP-enhanced aggregation of stirred human platelet rich plasma and the inhibition thereof by CN were monitored at 37 °C. It was found that preincubation for 1 minute of the platelet rich plasma (3 x $10^5$/$mm^3$) with 5 μl of the low molecular weight peak after Sephadex G-100 SF resulted in 76% inhibition of platelet aggregation induced by 10 μM ADP [Trikha et al. (1990), *supra*].

In a subsequent report it was noted that in crude venom, the inhibitor was not readily detectable due to the presence of platelet aggregating activity; however, following the first step of purification (hydrophobic interaction HPLC) inhibitory activity was separated from both aggregating activity and an α-chain degrading fibrinolytic enzyme present in the venom. Inhibitory activity was pooled following HPLC and applied to a hydroxylapatite HPLC column. In the final step of purification, $C_4$ reverse phase HPLC chromatography was employed. The yield of the homogeneous protein was 3-5 mg per gram of venom. CN was reported to have a molecular weight of 18-21 kDa under non-reducing conditions and 9 kDa under reducing conditions; thus, the molecule was believed to be a homodimer with the two subunits being held together by disulfide bond(s). Isoelectric focusing showed that the protein had an acidic pl. CN was reported not to exhibit fibrinolytic activity and was not a 5'-nucleotidase or a phospholipase based on molecular size and kinetics of inhibition of platelet aggregation. Following preincubation for 1 minute, CN at approximately 100 nM was reported to completely inhibit ADP-induced platelet aggregation [Trikha et al. (1990), *supra*].

It has further been reported that CN has 70 amino acids with five to six disulfide bridges, and that the sequence of CN appears to begin 10 amino acids downstream of applaggin (a platelet aggregation inhibitor from the venom of *Agkistrodon piscivorus piscivorus*). It was speculated that CN may have an insertion and/or a C-terminal extension of nine amino acids. It was further reported that a 50% inhibition ($IC_{50}$) of human platelet aggregation in platelet rich plasma was observed at 0.8 μg/ml of CN, and at 2.2 μg/ml with canine platelets [Trikha, M. et al., Journal of Cellular Biochem. 16F:180 (1992)].

CN was reported to inhibit binding of human fibrosarcoma (HT-1080) and c-Ha-ras transfected rat embryo (4R) cells to fibronectin coated plates but not to matrigel coated plates. Inhibition of 4R cell binding to fibronectin in the presence of CN at 1 μg/ml and 5 μg/ml was 46% and 88%, respectively, and for HT1080 cells inhibition was 89% and 85%, respectively [Trikha, M. et al., Proceedings of the American Association for Cancer Research 33:34 (1992)].

Since it appears that CN can inhibit interactions between integrins and their receptors, and may prove useful in the management of diseases associated with these interactions, there exists a need for improves methods to produce greater amounts of purified contortrostatin, substantially free from other snake venom components.

## SUMMARY OF THE INVENTION

The present invention fulfills the need for greater amounts of contortrostatin, which can be used to inhibit biological processes such as platelet aggregation, cell growth, adhesion, metastasis, and neovascularization. Native contortrostatin has been purified and a partial amino acid was determined by Edman degradation. This information enabled a cDNA cloning strategy, which resulted in a full-length cDNA sequence and a deduced amino acid sequence for a contortrostatin pre-cursor protein. The contortrostatin precursor includes a pro-protein region, a metalloproteinase region, and a disintegrin region. The metalloproteinase region includes a metal-binding motif and the disintegrin region includes an RGD loop, which can act as an integrin antagonist. Sequences for native contortrostatin are contained in the distintegrin region.

The present invention includes purified contortrostatin proteins, including the contortrostatin precursor, biologically active variants, and fragments thereof. The contortrostatin protein is in accordance with claim 1.

A purified contortrostatin, which can act as an integrin antagonist generally will include in each monomer a constrained Arg-Gly-Asp (RGD) sequence at the-tip of a flexible peptide loop of about 13 amino acid residues flanked by two Cys residues, such as the amino acid sequence comprising amino acid numbers 457 to 469 of SEQ ID NO: 2.

Biologically active variants, can include amino acid substitutions, deletions, and insertions, but will generally have an amino acid sequence that is at least 90% homologous to the pro-protein, metalloproteinase, disintegrin and/or contortrostatin regions of the precursor protein. Variants can also include a peptide recognized by an antibody to contortrostatin.

The proteins of the present Invention can be made using synthetic methods. The synthetic process can include transcribing and translating a contortrostatin cDNA molecule as disclosed herein, preferably within a transformed host cell. Alternatively, the process involves synthesizing a polypeptide having the amino acid sequence of contortrostatin, as disclosed herein.

Proteins prepared by recombinant DNA methodology will generally include the use of a recombinant DNA molecule comprising a DNA sequence coding on expression for contortrostatin, such as SEQ ID NO:1. Preferably the recombinant DNA molecule encodes sequences having at least one biological activity such as metalloproteinase (e.g. nucleotide numbers 657 to 1316 of SEQ ID NO: 1), or disintegrin (e.g., nucleotide numbers 1341 to 1535 of SEQ ID NO: 1). Moreover, the recombinant DNA molecule can also include pro-protein encoding sequences (e.g., nucleotide numbers 87 to 656 of SEQ ID NO: 1), the entire precursor protein (nucleotide numbers 87 to 1535 of SEQ ID NO: 1), or simply the sequences encoding native contortrostatin monomer (nucleotide numbers 1341 to 1535 of SEQ ID NO: 1).

The present invention further provides a vector, which includes the recombinant DNA molecule, that can be used to transform prokaryotic or eukaryotic host cells. Host cells can be mammalian cells, plant cells, insect cells, yeast and other fungi or bacteria. Processes for producing recombinant contortrostatin will generally include steps, such as culturing the host cell and recovering the contortrostatin expressed by the host cell.

The contortrostatin proteins of the present invention can be formulated as a pharmaceutically acceptable composition, comprising a pharmaceutically acceptable carrier and the purified protein. The pharmaceutical composition can then be used in a method of treating a patient having a disease associated with a ligand binding to an integrin receptor. The treatment generally involves administering the composition to the patient such that integrin binding to integrin receptors is substantially inhibited and the patient is treated. The method of treatment can be used to inhibit platelet aggregation, tumor metastasis, angiogenesis, neovascularization, cell adhesion, invasiveness, or growth.

## BRIEF DESCRIPTION OF THE DRAWINGS

The invention may be better understood with reference to the accompanying drawings, which include:

Fig. 1 illustrates the strategy for cloning contortrostatin cDNA, wherein Fig. 1A shows partial amino acid sequence of CN (CN) based on Edman Degradation Assay compared with other disintegrins illustrating common RGD sequences and highly conserved sequences, Fig. 1B shows the PCR primers, and Fig. 1C shows the principle of overlapping extension reaction used to generate the full-length cDNA of contortrostatin;

Fig. 2 shows electrophoresis of the PCR products, wherein a major band of about 1,300 bp was amplified with PCR primed by λgt10 forward and PCR-2 (lane 1), a band of approximately 700 bp resulted from PCR primed by PCR-2 and λgt 10 reverse (lane 2), and the overlapping product of the two fragments is shown in lane 3, with a molecular size of about 2,000 bp;

Fig. 3 shows the full-length nucleotide sequence of CN cDNA and the deduced amino acids, including an 86 nucleotide 5'-end non-translatable region (NTR), an open reading frame between nucleotides 87 and 1535, a termination codon at nucleotides 1536 to 1538, and a 3'-NTR, which includes an AATAAA site and ends with a poly-A tail;

Fig. 4 shows the multi-domain structure of contortrostatin precursor, compared with trigramin precursor and other snake venom hemorrhagic proteins;

Fig. 5 illustrates the formation of a contortrostatin homodimer, wherein Fig. 5A shows the amino acid sequence and disulfide bond pattern of the disintegrin kistrin compared to contortrostatin, which has a 6-amino-acid truncation at the N-terminus, including two half cystine residues resulting in two cysteine residues being unpaired, and Fig 5B shows the unpaired cysteines may participate in the formation of two intermolecular disulfide bonds to form a unique homodimeric structure, wherein the two monomers may be linked in a parallel or anti-parallel pattern;

Fig. 6 illustrates the results of determinations of CN inhibition of human, canine and rabbit platelet aggregation;

Figs. 7A and 7B illustrate the results of binding studies of CN to human (Fig. 7A) and canine (Fig. 7B) (GP)IIb/IIIa in the presence of a fixed saturating concentration of murine monoclonal antibody 7E3;

Fig. 8 is a schematic representation of an instrumented canine carotid artery showing placement of an ultrasonic flow probe, mechanical constrictor (stenosis) and intra-carotid anodal electrode for inducing intimal injury to the vessel wall to initiate thrombus formation;

Fig. 9 illustrates platelet counts and platelet aggregability as percent of the value at zero time in a canine treated with anisoylated plasminogen streptokinase activator complex (APSAC) and CN;

Fig. 10 shows CN inhibited adhesion of MDA-MB-435 to fibronectin;

Fig. 11 shows CN inhibited adhesion of DA-MB-435 to vitronectin;

Fig. 12 shows the inhibition of binding of human mammary carcinoma cells to immobilized CN with GRG-DSP;

Fig. 13 shows the inhibition of binding of human mammary carcinoma cells to immobilized CN with EDTA;

Fig. 14 shows the inhibition of invasion of MDA-MB-43 5 cells through a Matrigel coated invasion chamber;

Fig. 15 shows the effect of CN on the growth MDA-MB-435 tumor in experimental nude mice;

Fig. 16 is a photograph demonstrating tumor induced angiogenesis in a control chick embryo chorioallantoic membrane (CAM) (Fig. 16A), CAM treated with 20 μg of CN (Fig. 16B), and CAM treated with 150 μg of CN (Fig. 16B); and

Fig. 17 shows the effect of CN on the proliferation of MDA-MB-43 5 cells *in vitro.*

## DETAILED DESCRIPTION OF THE INVENTION

### Characterization, Cloning and Expression of CN

We have purified and characterized a disintegrin: contortrostatin (CN) from *A. c. contortrix* venom. CN is a homodimer with a mass of 13,505 for the intact protein and 6,956 for the reduced and pyridylethylated protein. To test binding affinity to platelet, (GP)IIb/IIIa (fibrinogen receptor), competition of CN with [$^{125}$1]7E3, an antibody directed to (GP)IIb/IIIA, was analyzed using human platelet rich plasma (PRP). CN displayed an $IC_{50}$ of 25 nM. Thus, CN is a potent β3 integrin antagonist.

The cDNA ofCN has been amplified from a library of *A. c. contortrix* venom gland cells, constructed in λgt10. Amino acid composition and partial amino acid sequences of CN have been determined by Edman degradation; see Fig. 1. Using this information a full-length cDNA of 2,027 nucleotides (SEQ ID NO:1), which encodes a contortrostatin precursor protein, has been cloned and sequenced.

As a member of disintegrin super family, CN shares high similarity with other disintegrins including, trigramin whose nucleotide sequences were known. Fig. 1A shows a partial amino acid sequence of CN based on Edman degradation assay. The partial sequence is also compared with other disintegrins as indicated. The RGD sequence is boldfaced. The highly conserved PCCDAATCKL sequences on which PCR primers are designed are underlined. Figs. 1A and 1B show how the cDNA of CN has been cloned by means of PCR using primers based on the highly homologous sequences among the disintegrin family as well as known λgt10 sequences flanking the cDNA inserts. The PCR primer pairs are: SEQ ID NO:5 (λgt10 forward primer) and SEQ ID NO:3 (PCR-1) 5'-GATTTACAGGTTGCAGCATCGC-3', which is antisense of trigramin cDNA encoding part of the underlined conserved sequence (Fig. 1A and 1B). SEQ ID NO:4 (PCR-2), which is complementary to PCR-1, and SEQ ID NO:6 (λgt 10 reverse primer). SEQ ID Nos. 5 and 3 amplify DNA coding for amino acids upstream to the underlined part. SEQ ID Nos. 4 and 6 amplify those coding for the downstream part of CN. Full length cDNA has been obtained by overlapping extension of the two pieces of PCR products (see Figs. 1C and 2).

The cDNA sequence and deduced amino acid sequence ofcontortrostatin precursor are shown in Fig. 3. The full length sequence is composed of 2,029 nucleotides (SEQ ID NO: 1). It is composed of an 86 nucleotide non-translatable region at the 5'end, an open reading frame coding for 483 amino acids (SEQ ID NO:2), and a 3' non-coding region.

Fig. 4 shows the multidomain structure of contortrostatin compared to that of four other snake venom hemorrhagic proteins: trigramin; Cat (catrocollastatin from *Crotalus atrox* venom); jararhagin (from *Bothrops jararaca* venom); and Ht-e (from *C. Atrox* venom). According to the structural division of snake venom metalloproteinases, the precursor ofcontortrostatin can be divided into a pro-protein (amino acid residues 1 to 190 of SEQ ID NO: 1 or 2), metalloproteinase (residues 191 to 410 of SEQ ID NO:1 or 2) and disintegrin (residues 419 to 483 of SEQ ID NO: 1 or 2) domains. The mature monomer of native disintegrin starts at D419, i.e., the aspartic acid residue at position 419. Underlined portions of Fig. 4 show the RGD sequences of both contortrostatin and trigramin, as well as the conserved HEMGHNLGIHH sequences of the zinc-binding motifs in the metalloproteinase domains of each molecule.

According to the present invention, therefore, there is also provided a precursor of contortrostatin having pro-protein, metalloproteinase, and disintegrin domains. This protein can be purified from natural sources such as snake venom or can be made by recombinant techniques as will be understood by those skilled in the art with reference to the disclosure herein.

Still further, the present application claims both the native and synthetic amino acid and nucleotide sequences. Unless otherwise modified, the term "protein" as used herein encompasses both native and synthetic polypeptides and peptides. Synthetic protein includes recombinant and chemically synthesized protein. Unless otherwise indicated, the term "contortrostatin" include both the native and synthetic versions of the proteins.

The term "nucleotide sequence" includes both the DNA and RNA sequences. For example, the nucleotide sequence for a contortrostatin protein ("contortrostatin nucleotide sequence") includes the gene ("contortrostatin gene") encoding the native and precursor protein, its complementary DNA, and the RNA corresponding to the foregoing; also included are messenger RNA encoding for the contortrostatin protein, its complementary RNA, and the DNA corresponding to the forgoing. Further, as used in this application the nucleotide sequences include: (1) the DNA sequences encoding the contortrostatin proteins, (2) the nucleotide sequences (which may be RNA or DNA) complementary to the foregoing sequences, (3) the corresponding RNA sequences to the DNA sequences wherein the Thymidine ("T") in the disclosed DNA sequences is replaced with Uracil ("U"), (4) nucleotide sequences wherein other nucleotides known in the art such as nucleotide analogs, replace those in the foregoing sequences, for example, 5-methyl-cytosine replacing cytosine, and (5) nucleotide sequences that are for example, within a 20% and preferably 10% variance to the foregoing nucleotide sequences.

Since nucleotide codons are redundant, also within the scope of this invention are equivalent nucleotide sequences which include: nucleotide sequences which code for or can be translated into the contortrostatin proteins, their protein variants, functional equivalents, or derivatives. These nucleotide sequences may also be used in the practice of the invention.

In addition to the above, contortrostatine nucleotide sequences also include: (1) nucleotide sequences that are capable of hybridizing to the coding sequences of the respective nucleotide sequences, under stringent hybridization conditions, and (2) fragments of or mutagenized nucleotide sequences of those disclosed herein which can encode or can be translated into proteins having substantially the same biological characteristics/activities of the respective contortrostatin proteins, e.g. integrin antagonist, zinc-binding, proteinase, anti-angiogenic factor, and further activities as disclosed herein in further embodiments and examples.

The terms "contortrostatin proteins", as used in relation to proteins include the respective proteins described in the Example section, below, and precursor proteins obtainable by the methods of the present invention, most preferably proteins exhibiting the properties of a disintegrin obtainable from the isolation methods of the Example section below, and: (1) protein variants of these proteins; *e.g.* these protein variants may contain amino acid sequences that have for example, at least 90% or more preferably at least 95% of their amino acids matching the sequences of the pro-protein, metalloproteinase, disintegrin and/or native contortrostatin regions of the protein; (2) the functional equivalents of these proteins and their variants, respectively; and (3) the derivatives, including fragments, of the contortrostatin proteins and their variants, respectively.

The variants can result from, *e.g.* substitution, insertion, or deletion of the amino acid sequences of the contortrostatin proteins. The derivatives of the proteins and their variants, include fragments of these proteins and immunoreactive peptides that specifically bind with antibody to contortrostatin.

Two amino acid sequences are functionally equivalent if they have substantially the same biological activities such as the ability to bind to integrin receptors. The proteins may be fused to other proteins, for example, signal sequence fusions may be employed in order to more expeditiously direct the secretion of recombinant contortrostatin proteins.

Substitutional variants of the proteins disclosed herein are those in which at least one residue in the disclosed sequences has been removed and a different residue inserted in its place. Preferably, the amino acid change is conservative. Thus, modifications of the contortrostatin proteins' primary amino acid sequences also include conservative variations. The term "conservative variation" as used herein denotes the replacement of an amino acid residue by another, biologically similar residue. Examples of conservative variations include the substitution of one hydrophobic residue such as isoleucine, valine, leucine or methionine for another, or the substitution of one polar residue for another, such as the substitution of arginine for lysine, glutamic for aspartic acid, or glutamine for asparagine, and the like. The term "conservative variation" also includes the use of a substituted amino acid in place of an unsubstituted parent amino acid provided that the polypeptide retains its biological activity, *e.g.*, antibodies raised to the substituted polypeptide also immunoreact with the unsubstituted polypeptide.

Further, as is the case for all proteins, the precise chemical structure depends on a number of factors. As ionizable amino and carboxyl groups are present in the molecule, a particular protein may be obtained as an acidic or basic salt, or in neutral form. All such preparations which retain their activity when placed in suitable environmental conditions are included in the definition. Additionally, the primary amino acid sequence may be augmented by derivatization using sugar moieties (glycosylation) or by other supplementary molecules such as lipids, phosphate, acetyl groups and the like, more commonly by conjugation with saccharides. The primary amino acid structure may also aggregate to form complexes, most frequently dimers. Certain aspects of such augmentation are accomplished through post-translational processing systems of the producing host; other such modifications may be introduced *in vitro.* In any event, such modifications are included in the definition so long as the activity of the protein is not destroyed. It is expected that such modifications may quantitatively or qualitatively affect, the activity, either by enhancing or diminishing the activity of the protein in various assays.

Individual amino acid residues in the chain may also be modified by oxidation, reduction, or other derivatization, and the protein may be cleaved to obtain fragments which retain activity. Such alterations which do not destroy activity do not remove the protein sequence from the definition. The following discusses some of the modifications in further detail by way of example.

Thus, glycosylation variants are included within the scope of contortrostatin proteins. They include variants completely lacking in glycosylation (unglycosylated) and variants having at least one less glycosylated site than the native form (deglycosylated) as well as variants in which the glycosylation has been changed.

As illustrated in the examples, native CN may be isolated from the venom of *Agkistrodon contortrix contortrix* in a relatively straightforward manner. Alternatively, CN may also be prepared by exploiting a variety of biochemical methods in current use, such as recombinant DNA technology or the like. Moreover, the sequence information reported herein can be used for making probes to identify variants, fragments, conserved domains or pro-proteins having substantial homology to CN and its precursor(s). Once identified, the genes may be isolated, further manipulated, and cloned into expression vectors.

There is also provided a vector containing a DNA molecule encoding a contortrostatin protein made according to techniques understood by those with skill in the art with reference to the disclosure herein. In the present invention, the contortrostatin nucleotide sequence may be inserted into a recombinant expression vector. The term "recombinant expression vector" refers to a plasmid, virus or other vehicle known in the art that has been manipulated by insertion or incorporation of a contortrostatin genetic sequence. Such expression vectors contain a promotor sequence which facilitates the efficient transcription of the inserted genetic sequence in the host. The expression vector typically contains an origin of replication, a promoter, as well as specific genes which allow phenotypic selection of the transformed cells. These vectors may be used to transform competent hosts to produce transformants that are capable of producing the snake venom protein.

Further, there is provided, a prokaryotic or eukaryotic host cell stably transformed or transfected by the vector, as well as a method of making the contortrostatin protein or its biological variants. The method includes the steps of, first culturing a prokaryotic or eukaryotic host cell transformed with DNA encoding for contortrostatin protein; and then, recovering the contortrostatin protein. The host cell can be mammalian cells, plant cells, insect cells, yeast and other fungi, or bacteria.

Sources of DNA sequences encoding for the proteins include isolated DNA from suitable cells or cell lines, cloned DNA from a snake venom genomic library or cloned DNA from a complementary DNA library, where the total complementary DNA is reverse transcribed to DNA and cloned. Once a DNA sequence is identified which encodes for a protein of interest, the sequence of bases may be determined by known means [e.g., Maxam and Gilbert, Proc. Natl. Acad Sci. USA 74:560 (1977)]. In addition, hybrid DNA technology may be employed for obtaining expression. The DNA sequence may be restriction mapped and appropriate sites for cleavage defined. In this way, the sequence may be excised and introduced into a vector having the appropriate regulatory signals. A more detailed discussion of suitable techniques for identification and expression of disintegrin genes is provided in, e.g., U.S. Patent Nos. 5,182,260 and 5,196,403 to Maraganore et al., the entire disclosures of which are hereby incorporated by reference.

Further, the sequence encoding the native protein may then be manipulated (for example, by single or multiple mutations or deletions) in a manner well known in the art to provide modified proteins, in which changes of one or more amino acids have been introduced. Following the procedures described herein, the determination of whether a particular polypeptide exhibits an activity profile characteristic of CN would then be a matter of routine experimentation. Accordingly, the present invention contemplates both the native CN and mutations thereof which exhibit the characteristic activity profile defined herein. Moreover, CN may also be employed in the form of a fusion protein with a suitable thrombolytic agent. Fusion proteins of this type may be prepared in a manner analogous to that described for formation of platelet aggregation inhibitor/anti-thrombin polypeptide fusion proteins in the aforementioned U.S. Patent Nos. 5,182,260 and 5,196,403 to Maraganore et al.

Despite the high sequence homology with other disintegrins (shown in Fig. 1A) [Niewiarowski, S., McLane, M. A., Kloczewiak, M., and Stewart, G. J. Disintegrins and Other Naturally Occurring Antagonists of Platelet Fibrinogen Receptors, Seminars in Hematology. 31:289-300 (1994)], contortrostatin displays a unique amino-terminal sequence with a truncation of about 8 amino acids. In comparison with other disintegrins, two cysteine residues are missing at the amino-terminus of contortrostatin. It is well established that monomeric disintegrins possess an even number ofcysteines, all of which are involved in disulfide bond formation. The missing two cysteines in the contortrostatin precursor may lead to disruption of disulfide bond pairing. This in turn may lead to the formation of two intermolecular disulfide bonds between two identical chains to form the homodimeric structure of contortrostatin. Fig. 5 presents a hypothetical model of the structure of the dimeric disintegrin as compared to a disintegrin, kistrin, whose disulfide bond positioning is known [Adler, M., Carter, P., Lazarus, R. A., and Wagner, G. Cysteine pairing in the glycoprotein IIb/IIIa antagonist kistrin using NMR, chemical analysis, and structure calculations, Biochemistry 32:282-9 (1993)]. In Fig. 5A, the sequences of the two disintegrins are compared. It can be readily seen that kistrin, with 12 half-cystines, forms 6 disulfide bonds. By comparison, contortrostatin, which has two unpartnered cysteine residues, can form the homodimeric structures presented in Fig. 5B. We are not sure at present which alignment is preferred, but we are confident, based on mass spectral analysis and SDS-PAGE of native and reduced contortrostatin, that a homodimeric structure most probably identical to one of the two shown, is formed.

CN is clearly quite different from applaggin, as the latter has been demonstrated unmistakably to be a monomer [Wencel-Drake et al. (1993), *supra*]. Moreover, CN does not inhibit platelet release reactions, as has been demonstrated to be the case with applaggin in the aforementioned U.S. Patent Nos. 5,182,260 and 5,196,403 to Maraganore et al. Finally, despite the similarities in sequence there are also significant differences between the sequences with respect to both the start site and the ensuing sequences.

## Methods of Use

CN has been found to be a potent inhibitor of human, rabbit and canine platelet aggregation *in vitro.* Unlike applaggin, however, CN does not inhibit platelet release reactions. Platelets comprise a plurality of different granules, including alpha granules and dense granules, whose contents are released when the platelets aggregate. The finding that CN does not inhibit platelet release of granule contents (including ATP from the dense granules) signifies that the platelets may still release their contents (and thus maintain some semblance of normal physiological activity) notwithstanding the inhibition, of aggregation. By contrast, when applaggin inhibits platelet aggregation it also inhibits platelet release (as measured by, e.g., inhibition of serotonin release from the dense granules). Thus, normal platelet physiological processes are necessarily further perturbed with administration of applaggin.

Several lines of evidence indicate that CN inhibits platelet aggregation by binding specifically to the (GP)IIb/IIIa integrin receptor. For example, in a fibrinogen-(GP)IIb/IIIa ELISA [Dennis, M.S. et al., Proc. Natl. Acad Sci. (USA) 87:2471 (1990)], in which the extent of purified (GP)IIb/IIIa bound to immobilized fibrinogen can be quantitated, CN effectively blocks (GP)IIb/IIIa binding. Additionally, the partial amino acid sequence of CN indicates considerable similarity with other disintegrins known to bind to (GP)IIb/IIIa. Finally, CN blocks 7E3 binding to (GP)IIb/IIIa. 7E3 is a murine monoclonal antibody that specifically binds to (GP)IIb/IIIa, thereby inhibiting human and canine platelet aggregation [Coller, B.S. et al., J. Clin. Invest. 72:325 (983)]. In the presence of a low concentration of CN, 7E3 binding to platelets is significantly inhibited.

Three snake venom disintegrins, kistrin [Yasuda et al. (1990), *supra*], echistatin [Holahan et al. (1991), *supra*] and bitistatin [Shebuski, R.J. et al. (1990), *supra*], have demonstrated a potential role as antithrombotic agents for use in thrombolytic therapy by enhancing and sustaining arterial thrombolysis in conjunction with recombinant tissue plasminogen activator. Based on the low $IC_{50}$ values of CN, its *in vivo* efficacy as an antithrombotic agent has been examined. Using a canine reoccluding carotid arterial thrombosis model, CN has been found to efficiently sustain opening of the carotid artery in conjunction with anisoylated plasminogen streptokinase activator complex (APSAC). APSAC alone was found insufficient to prevent the rapid reocclusion of the carotid artery. Heparin was not needed to sustain opening when CN was administered with APSAC. This is a significant distinction over other disintegrins (e.g., echistatin, bitistatin and kistrin) which have been evaluated in models of coronary artery thrombosis. ,

The compositions of the present invention are particularly useful for treatment of thrombotic diseases in mammals, alone or in conjunction with one or more thrombolytic agents. In particular, the compositions of the present invention have utility in treating or preventing arterial, venous and microvascular thrombosis and thromboembolism. Thus, the compositions have utility in treating stroke, transient ischemic attacks, arteriosclerosis, atherosclerosis, pulmonary embolism, aneurisms and angina. In particular, the compositions have utility in preventing or treating myocardial infarctions.

The compositions of the present invention also have utility in inhibiting metastasis in melanoma, carcinoma and sarcoma patients. CN has been observed to bind to at least two sites on human melanoma M24met cells: a high affinity site with a dissociation constant (Kd) of 1.1 ($\pm$ 0.7) nM and 96,000 ($\pm$ 39,000) sites per cell; and a lower affinity site with a Kd of 41 ($\pm$ 13) nM and 480,000 ($\pm$ 90,000) sites per cell. Moreover, CN has been found to inhibit human melanoma M24met cell adhesion to fibronectin and vitronectin, and to a lesser extent to collagen and laminin. Thus, methods and compositions for preventing metastases in melanoma, carcinoma and sarcoma patients are also contemplated as within the scope of the present invention.

In accordance with the present invention, the unique properties of disintegrins are exploited in methods and compositions for preventing metastasis in carcinoma, sarcoma and melanoma patients. In particular embodiments the positions and methods are provided for preventing metastasis in breast cancer patients.

The protein contortrostatin from southern copperhead snake yenom possesses potent anti-tumor activity. A sophisticated technique has been developed to purify this protein from the complex mixture of proteins found in southern copperhead venom. As indicated above, originally CN was characterized as an inhibitor of platelet aggregation. We have purified several disintegrins from snake venoms. Contortrostatin, (CN) was purified from southern copperhead venom. Disintegrins contain a constrained Arg-Gly-Asp (RGD) sequence at the tip of a flexible peptide loop, of about 13 amino acid residues flanked by Cys residues, protruding from the main protein core. See, e.g. amino acid residues 457 to 469 of SEQ ID NO: 1 or 2. This exposed RGD sequence enables disintegrins to bind to integrins with high affinity.

We have developed a metastatic breast cancer model by implanting human breast cancer cells into the mammary fat pads of mice. The mice we use were genetically manipulated so that their immune system is deficient and they are unable to reject the implanted human cancer cells. We observed that palpable tumor masses developed in the mammary fat pads two weeks after cancer cell implantation, and that tumor cells spread to the lungs in untreated animals within 12 weeks. CN or placebo was injected daily into tumors in several different groups of mice. Following treatment we found that the size of the tumor masses in the CN treated mice were significantly smaller than those in placebo-treated mice. Significantly, the CN-treated group showed >90% inhibition of tumor spread to other sites in the body (metastasis), as compared to the placebo group. Our studies indicate that CN blocks the attachment of breast cancer cells to proteins which are essential components of blood vessel walls. CN also inhibited new blood vessel formation (neovascularization) induced by breast cancer cells following incubation on a chick embryo membranous respiratory organ called the chorioallantoic membrane while placebo treatment did not. Since neovascularization is critical to continued proliferation of a growing tumor the ability to inhibit the growth of new vessels is an important anti-cancer action of CN.

Based on these studies it appears that disintegrins such as the snake venom protein contortrostatin possess anti-metastatic activity. Our findings suggest that CN blocks several critical steps in metastasis and is, therefore, more potent than other agents which only block a single step.

Disintegrin-containing compositions are also useful in treatment of osteoporosis. Osteoclasts are multinucleated cells up to 400 μm in diameter which resorb mineralized tissue in vertebrates. Bone resorption appears to proceed by a combination of processes involving attachment to bone, polarized secretion of acid and proteases, and active motility of osteoclasts along the bone substrate; osteoclasts bind to bone via an RGD-sequence as an obligatory step in bone resorption, and this RGD-binding integrin is at adhesion structures [Sato, M. et al., J. Cell Biol. 111:1713(1990)]. The molecular mechanisms whereby osteoclasts attach to bone are not well understood; however, by analogy to other cells, members of the integrin superfamily of divalent cation-dependent adhesion molecules are believed to mediate this interaction Disintegrins, such as echistatin [Sato et al. (1990), *supra*] and presumably CN, inhibit bone resorption by isolated osteoclasts; the mechanism of action is presumably by disrupting adhesion structures.

Finally, CN has utility in the promotion of wound healing. Events involved in wound healing are known to include alterations in integrin expression or functional activity and suggest that integrin receptor modulation plays a central role in wound repair and inflammation. Fibronectin is also known to play a number of roles in the wound healing process. Although fibronectin function is thought to be critical to effective wound healing, there are reports that at least one of its activities (the binding of bacteria) may be counterproductive [Grinnell, F., J. Cell. Biochem. 26:107 (1984); Clark, R.A.F., Arch. Dermatol. 124:201 (1988)]; the presence of fibronectin in the wound bed may thus promote bacterial attachment and infection. Fibronectin also appears to be intimately involved in keloid formation. Keloids are a pathological consequence of wound healing that affects a significant proportion of non-Caucasian patients. Keloids are benign tumors of connective tissue that grow beyond the boundary of the original wound and are rich in fibronectin and type I collagen [Sible, J.C. & Oliver, N., J. Cell. Biochem. Suppl. 16F:170 (1992)]. By virtue of their inhibition of cell-cell and cell-extracellular matrix interactions (including interaction with fibronectin), disintegrins such as CN would be expected to have a profound effect on processes involved in wound repair, including keloid formation.

A major problem following obstetrical and gynecological surgery is the formation of adhesions. This widespread phenomenon observed in peritoneal wound repair is a leading cause of pain, intestinal obstruction and infertility. Adhesion formation appears to involve an imbalance in the fibrinolytic and fibroproliferative inflammatory responses and may also involve a modulation of the cell-cell or cell-extracellular matrix interactions. There is strong evidence for an important role of fibrin during the initial stages of adhesion formation [diZerega, G.S., Prog. Clin. Biol. Res. 381:1 (1993)]. The presence of cellular elements, including platelets, further exacerbates the role of fibrin. In view of the role of platelets and fibrin in adhesion formation, the use of disintegrins such as CN as a potential therapeutic agent is most attractive.

In preliminary studies in a rabbit model of adhesion formation, abrasion and devascularization of the uterine horns of rabbits were employed to induce adhesion formation during wound healing in untreated animals [Rodgers, K. et al., Int. J. Fertil. 35:40 (1990)]. Alzet pumps were employed to continuously deliver CN at a rate of 10 μl/hr (36 μg/ml). In this model system, decreased adhesion formation was observed in treated animals compared to controls.

Useful compositions comprising CN comprise at a minimum an amount of CN effective to achieve the desired effect (i.e., prevent thrombus formation, prevent metastasis in carcinoma patients, prevent adhesion formation, etc.) and a suitable carrier or excipient. Generally, in these compositions, CN is present in an amount sufficient to provide about 0.01 mg/kg to about 50 mg/kg per day, preferably about 0.1 mg/kg to about 5.0 mg/kg per day, and most preferably about 0.1 mg/kg to about 0.5 mg/kg per day. Such compositions have particular utility in the prevention of thrombus formation.

Alternatively, CN is administered in combination with at least one thrombolytic agent present in an amount effective to achieve thrombolysis. Suitable thrombolytic agents include, but are not limited to, the following: anisoylated plasminogen streptokinase activator complex (APSAC); tissue-type plasminogen activator (tPA); urokinase-type plasminogen activator (uPA); and fibrolase, a snake venom fibrinolytic agent as described in U.S. Patent 4,610,879 to Markland, Jr. et al.

CN may be administered by a variety of heretofore known means suitable for delivery thereof into the blood stream in substantial amounts. Intravenous administration of CN in a suitable liquid vehicle or excipient is presently contemplated as the preferred route of administration. CN is soluble in water, and may therefore be effectively administered in a suitable aqueous solution (e.g., phosphate buffered saline). Alternatively, CN may be administered orally (in the form of tablets or capsules formulated with a suitable binder or excipient material, or in the form of aqueous or oily suspensions, solutions, emulsions, syrups or elixirs) or as a parenteral suspension. As is well known in the art, adjuvants such as local anesthetics, preservatives, buffering agents, lubricant, wetting agents, colorants, flavorings, fillers and diluents may suitably be included in any of these formulations.

## Reference EXAMPLES

The accompanying examples are intended for purposes of illustration only.

In conducting the following examples, lyophilized venom from *Agkistrodon contortrix contortrix* was obtained from Biotoxins, Inc., St. Cloud, FL. All chemicals were of the highest grade available. Pierce protein assay kit using bicinchoninic acid was employed to determine protein concentrations [Smith, P.K. et al., Anal. Biochem. 150:76 (1985)].

For hydrophobic interaction (HIC)-HPLC a Perkin Elmer 410 LC pump was employed with a LC-95 UV/VIS detector. For reverse phase HPLC a Spectra Physics LC 8810 pump was employed with an SP 8450 UV/VIS detector. Absorbance for HIC-HPLC was monitored at 280 nm and for RP-HPLC at 215 nm. A polypropyl aspartamide (250 x 21 mm) column (Poly LC, Columbia, MD) was used for hydrophobic interaction HPLC. C 18 (218TP54 and 218TP510) columns were used for reverse phase (RP) HPLC (Vydac, Hesperia, CA). For cation exchange chromatography a CM (carboxymethyl) 300 column (SynChrom, Inc., Lafayette, IN) was employed.

## Example 1

### Purification and characterization of CN

CN was purified from *Agkistrodon contortrix contortrix* (Southern copperhead) venom using a four step HPLC procedure. For the first step of purification crude venom (1 g) was dissolved in 0.1 M phosphate buffer containing 1 M ammonium sulphate, pH 6.8 (buffer A) and applied to the polypropyl aspartamide HIC-HPLC column. Elution was achieved as follows: 50 minutes isocratically with 100% buffer A; a linear gradient for 90 minutes to 0.1 M phosphate, pH 6.8 (buffer B); 40 minutes isocratic at 100% buffer B. Fractions of 10 ml were collected in a Pharmacia Frac 100 fraction collector at 4° C using a flow rate of 5 ml/min. Fractions containing platelet aggregation inhibiting activity were pooled and concentrated by ultra-filtration using an Amicon stir cell with a YM3 membrane. Proteins were detected at 280 nm; platelet aggregation inhibiting activity was observed in the flow through.

Further purification was achieved by C18 RP-HPLC. Fractions containing platelet aggregation inhibiting activity were pooled and concentrated for this second step. The C18 column (218TP510) was equilibrated with 95% of 0.1% TFA in water (solvent A) and 5% of 80% acetonitrile in 0.1 % TFA in water (solvent B). Elution was achieved as follows: isocratic at 95% solvent A and 5% solvent B for 10 minutes; a linear gradient to 40% solvent B in 65 minutes; linear gradient to 100% solvent B in 20 minutes; isocratic at 100% solvent B for 25 minutes. Fractions were collected manually every minute at a flow rate of 7 ml/minute. CN eluted at 28% acetonitrile (66 minutes).

Fractions containing platelet aggregation inhibiting activity were pooled and rerun on the same C 18 RP-HPLC column using a shallower gradient. Elution was achieved as follows: isocratic at 80% solvent A and 20% solvent B for 20 minutes; a linear gradient to 30% solvent B over 90 minutes; and a 25 minute linear gradient to 100% solvent B. CN eluted as a sharp peak at 22% acetonitrile (82 minutes). The minor peak eluting just before CN also contained platelet aggregation inhibiting activity and had a similar molecular weight to that of CN; due to the low yield, this peak was not further characterized.

A final purification step was performed using pooled fractions from the previous step. These pooled fractions were applied to a cation exchange, CM300, HPLC column and elution was achieved by an increasing gradient of sodium chloride. CN elutes at 52.5 minutes (160 mM NaCl). This step achieved a separation of CN from isoforms thereof. Yields of 1-2 mg of the four-step purified CN were obtained per gram of crude venom.

For SDS-polyacrylamide gel electrophoresis (SDS-PAGE) Tris-Tricine 16.5% gel was used according to published protocols under reducing and non-reducing conditions [Schagger, H. & Von Jagow, G., Anal. Biochem. 166:368 (1987)]. The gel was run using a BioRad minigel system and stained with silver [Morrisey, J.H., Anal. Biochem. 117:307 (1981)] or Coomassie blue R250.

SDS-PAGE analysis of CN revealed that it has a molecular mass of approximately 15,000 Daltons under non-reducing conditions and 5,000-7,000 Daltons under reducing conditions. This strongly suggests that CN is composed of two subunits. Another possibility, albeit unlikely, is that the large difference in migration may be attributed to differential uptake of SDS under non-reducing and reducing conditions.

The molecular weight of CN was confirmed by mass spectrometry using a triple quadrupole instrument with an electrospray ion source. A mass of 13,507 Daltons was determined for intact CN; the analysis also indicated a high degree of purity. Mass spectrometry of the reduced and pyridylethylated protein gave a mass of 7,996 Daltons. This is the expected value for the individual chains of a homodimer of this molecular weight, taking into account the incorporation of 1,248 mass units for the 12 pyridylethyl groups incorporated into the 6 reduced disulfide bounds (based on homology with known disintegrins, there should be 6 disulfide bonds). These findings place CN in a unique position among all the disintegrins reported to date in that it exists as a dimer. Scatchard analysis of CN binding to unactivated human platelets revealed a single class of binding sites with a dissociation constant ($k_d$) of 37 nM and number of binding sites ($B_m$) equal to 100,000. Reduction of the disulfide bonds completely eliminated platelet aggregation inhibitory activity, even at concentrations ten times the $IC_{50}$, suggesting that structural parameters are critical for maintaining activity.

## Example 2

### cDNA Cloning of Contortrostatin Using Polymerase Chain Reaction

Partial amino acid sequence analysis of contortrostatin using Edman degradation methods suggested that the subunit of contortrostatin is homologous with other disintegrins (Fig. 1A) with the cysteine residues aligned, as well as the RGD sequences [Niewiarowski, S., McLane, M. A., Kloczewiak, M., and Stewart, G. J. Disintegrins and Other Naturally Occurring Antagonists of Platelet Fibrinogen Receptors, Seminars in Hematology 31: 289-300 (1994)]. The strategy for cloning contortrostatin cDNA with PCR is based on the structural homology among the disintegrin family. Design of the PCR primers is schematically illustrated in Fig 1. The underlined sequences in Fig. 1A are highly conserved among disintegrin family. PCR primers were synthesized based on this region. The nucleotide sequence coding this region in the cDNA of trigramin from *Trimeresurus gramineus* was used to synthesize the primers PCR-1 and PCR-2 (Fig. 1B). PCR-1 and PCR-2 are complimentary primers corresponding to the coding sequence of a consensus sequence PCCDAATCKL among disintegrins. Their nucleotide sequences are:

PCR-1: 5'-GTTTACAGGTTGCAGCATCGC-3' (SEQ ID NO: 3)
PCR-2: 5'-GCGATGCTGCAACCTGTAAAC-3' (SEQ ID NO: 4)

λeg10 forward and reverse primers which flanks the EcoRI site of the vector were used for PCR. Nucleotide sequences of the primers are listed below:

λgt10 forward primer: 5'-AGCAAGTTCAGCCTGGTTAAG-3' (SEQ ID NO:8)
λgt 10 reverse primer: 5'-CTTATGAGTATTTCTTCCAGGGTA-3' (SEQ ID NO: 6)

Oligonucleotide primers were synthesized by the Microchemical Core Facility of the University of Southern California Comprehensive Cancer Center. Primers were provided in a deprotected lyophilized form, and were resuspended and diluted to the appropriate concentration with water prior to use.

## Example 3

### PCR amplification of contortrostatin cDNA

We used the cDNA library of *Agkistrodon contortrix contortrix* venom gland constructed in λgt 10 vector at the EcoRI site. The estimated titer of the library was $10^{10}$ plaque-forming units (pfu)/ml and the complexity was 50,000. 500μl of the cDNA library phage solution was mixed with 500μl of 20% polyethylene glycol (PEG)/ 1M NaCl solution in an Eppendorf tube. The Eppendorf tube was inverted twice and incubated at room temperature for 30 min. The solution was then centrifuged at 14,000 rpm for 10 min. The supernatant was discarded and the pellet was resuspended in 100 μl of sterile water. The suspension was incubated with 10μl of proteinase K (10 mg/ml) at 50°C for 1 hr. The phage particle suspension was extracted with phenol/chloroform twice and the DNA was precipitated with 1/10 volume of 3M sodium acetate and 2 volumes of absolute ethanol followed by washing with 80% ethanol. DNA was resuspended in 10μl of sterile water in preparation for PCR.

The PCR reaction was set up as follows: 5μl of DNA solution was mixed with 1 μl of 25 mM dNTP's (Pharmacia), 1 μl (100 ng/μl) each of forward and reverse PCR primers, and 5 μl of 10x PCR buffer. The final volume of the reaction was brought up to 50 μl with water. After mixing, one drop of mineral oil was applied on top of the liquid. The Eppendorf tubes were pre-heated to 98°C for 5 min, then incubated at 70°C and 60°C for 1 min respectively before cooled down to room temperature. 2.5 units of Taq DNA polymerase (Pharmacia) was added to each mix. The thermal cycler was programmed as follows: 96°C for 15 sec, 55°C for 30 sec, 72°C for 1 min. PCR amplification was performed for 30 cycles. Following the last cycle, a final 72°C extension step for 7 min was allowed before the samples were cooled down to 30°C. The PCR product was extracted with chloroform before it was analyzed by agarose gel electrophoresis. The bands resolved by electrophoresis were recovered from the agarose gel using Geneclean kit (Bio 101, Inc.) according to the manufacturer's manual for direct DNA sequencing.

λgt10 forward and PCR-1 primers were used to amplify the up stream region of the adhesion site (Fig 1B). Similarly, PCR-2 and λgt10 reverse primers were coupled to amplify the down stream part of the cDNA (Fig. 1B). A major band of about 1300 bp (designated CN-N) was obtained with the first pair of primers (Fig. 2, lane 1), and a major band of approximately 700 bp (designated CN-C) resulted from PCR using the later pair of primers (Fig. 2, lane 2). CN-N and CN-C were subjected to nucleotide sequencing analysis prior to the overlapping extension. As expected, CN-N demonstrated high similarity to cDNA of trigramin which encodes its N-terminal signal peptide. CN-C nucleotide deduced amino acid sequence was very similar to the disintegrin COOH-terminal sequence encoding the ROD site.

Since PCR-1 and PCR-2 were complimentary, CN-N and CN-C overlap at this site, and therefore, can be assembled into a full length cDNA. To accomplish this goal, we used an overlapping extension method as shown in Fig. 1C. Briefly, equal molar amount of double-strand PCR products CN-N and CN-C were mixed with λgt 10 forward and reverse primers. After denaturation of both double-strands, the subsequent reannealing results in two kinds of molecules. One is annealed CN-N and CN-C at the adhesion site with the recessive ends as 3'-ends. This molecule can be automatically elongated into full length double-strands using PCR. The other molecule is similarly annealed, but with the recessive ends as 5'-ends. Although these molecules are not self-elongated, the recessive parts can be filled in with priming by λgt10 primers at each end. Fig. 2, lane 3 shows the products of overlapping extension as resolved by agarose gel electrophoresis. The size of the major band is estimated to be 2,000 bp which equals the sum of CN-N and CN-C, and is thus designated "full length". The full length band was recovered from the gel and treated with EcoRI. Subsequently, this piece of DNA was subcloned into the plasmid vector pcDNA3.1 (+).

## Example 4

### Subcloning of PCR product into plasmid vector

Plasmid pcDNA3.1(+) was digested with EcoRI (Pharmacia) followed by dephosphorylation using T4 phosphatase (Boehringer-Mannheim). PCR overlapping extension product was also digested with EcoRI. The PCR product was inserted into the linearized vector by ligation reaction use, T4 ligase (Pharmacia) at 16°C overnight. All the reactions were set up and performed according to standard protocols. Successful ligation was selected by plating transformed *E. coli* (DH5α) on ampicillin containing plates. Plasmids containing the insert were amplified in *E. coli.* Purified plasmid DNA was obtained with Qiagene DNA Miniprep columns.

## Example 5

### DNA sequencing

Automated DNA sequencing was performed by the Microchemical Core Facility. PCR primers were used as sequencing primers for direct sequencing of PCR products. For sequence analysis of the insert in plasmid pcDNA3.1 (+), T7 promoter primer and BGH reverse primer, which flanks the multiple cloning sequence (MCS), was utilized to initiate the assay. Typical reactions gave readable sequences of 400 to 600 bp. The sequencing reactions were performed on double-stranded DNA in the case of plasmid DNA. With the synthesis of new sequencing primers, additional sequences were obtained, and these were assembled into overlapping contiguous sequences using DNAsis computer program.

Fig. 3 shows the full length nucleotide sequence inserted between the EcoRI sites. It is composed of 2,029 nucleotides, which is the size of the full length band of the overlapping extension (Fig. 2, lane 3). Following an 86-nucleotides 5'-end non-translated region (5'-NTR), an open reading frame is found between nucleotides number 87 and 1535. Nucleotide 1536 to 1538 is the termination codon. The 3'-NRT possesses an AATAAA site in the 3'-end noncoding region, and ends with poly(A) tail, suggesting that the cDNA we obtained with the overlapping extension was indeed a complete cDNA (Fig. 3). The open reading frame encodes 483 amino acids. The structure of cDNA deduced amino acid sequence can be divided into three domains. The first 190 amino acids, starts with methionine, are highly similar to the pro-protein of many cloned snake venom proteins (comparison is shown in Fig. 4). From amino acid 191 to 418 is the metalloproteinase domain including a Zinc-binding motif HEMGHNLGISH (aa 334 to 344). The remaining 65 amino acids belong to contortrostatin monomer which is identical to the known partial amino acid sequence of contortrostatin determined by Edman methodology. This sequence is very similar to those of many disintegrins whose sequences have been determined (Fig. 4 and 1A). The calculated molecular weight of the disintegrin is 6.77 kDa, which is equal to that of CN monomer. The RGD (aa 461 to 463) sequence is in bold face letters. The three-domain structure matches the precursor model of snake venom metalloproteinase and disintegrin proposed by Kini et al. [Structural Domains in Venom Proteins: Evidence that Metalloproteinases and Nonenzymatic Platelet Aggregation Inhibitors (Disintegrins) from Snake Venoms are Derived by Proteolysis from a Common Precursor, Toxicon 30:265-296, (1992)]. There is evidence that disintegrins are synthesized in the snake venom gland cells as a multi-domain precursor which undergo post-translational proteolysis and folding to generate a mature disintegrin.

**Example 6**

**Assay of platelet aggregation inhibitory activity**

Column fractions obtained during purification were assayed for activity using fresh human platelet rich plasma (PRP) prepared from blood obtained from human volunteers who had had no medication for at least two weeks. Blood (36 ml) was drawn into 4 ml of 0.1 M citrate and centrifuged at 150 x g for 20 minutes. The supernatant, PRP, was removed and the remaining blood was centrifuged at 10,000 RPM to obtain platelet poor plasma (PPP). Platelet counts were adjusted to 250,000 platelets/µl using a Coulter counter. A Helena four channel aggregometer was used to monitor platelet aggregation. Inhibition of ADP-induced platelet aggregation was monitored at 37°C by adding venom fractions one minute prior to the addition of ADP (10-20 µM final concentration). Fractions exhibiting platelet aggregation inhibiting activity were pooled and further purified. Rabbit and canine PRP was prepared by the same procedure and used in the studies described below.

CN inhibited ADP-induced platelet aggregation in human, canine, and rabbit PRPs (Fig. 6). Empty circles represent human platelet rich plasma, solid circles represent canine PRP, and empty triangles represent rabbit PRP. Varying concentration of CN were preincubated for one minute with PRP prior to the addition of ADP. CN (0.73 µg/ml) inhibited 10 µM ADP-induced human platelet aggregation by 50% ($IC_{50}$). The $IC_{50}$ for 20 µM ADP-induced canine platelet aggregation was 1.8 µg/ml for CN. Interestingly, the $IC_{50}$ for CN mediated inhibition of rabbit platelet aggregation was considerably higher; the $IC_{50}$ for 20 µM ADP-induced rabbit platelet aggregation was 17.3 µg/ml for CN.

Example 7

**Measurement of (GP)IIIb/IIIa specific binding**

Measurement of CN binding to platelet (GP)IIb/IIIa receptor was carried out using PRP prepared from blood obtained from human volunteers or male mongrel dogs. PRP was prepared as described above and the platelet count was determined with a H-10 cell counter (Texas International Laboratories, Inc., Houston, TX). PRP (180 µl) was incubated with 20 µl of varying concentrations ofCN at room temperatures. Radiolabelled antibody ($^{125}$I-7E3 IgG, 20 µl, 18 mg/ml, 80,000 cpm), specific for (GP)IIb/IIIa, was then added and the mixture incubated for 30 minutes. To establish equilibrium binding, 50 µl aliquots of the binding assay mixture were layered over 200 µl of 30% sucrose in 0.4 ml microcentrifuge tubes and spun at 10,000 RPM for 4 minutes in a swinging bucket rotor to separate platelet-bound antibody from free antibody. The pellet and the supernatant were separated and counted in a Packard Minaxi 5000 series gamma counter. The number of molecules of $^{125}$I-7E3 bound per platelet in the presence and absence of CN was calculated by using the following formula:

$$\frac{(4) \times 0.9\ \mu g\ 7E3 \times 3.76 \times 10^{12}\ \text{molecules}\ 7E3/\mu g}{(5)}$$

wherein (1) = Pellet counts; (2) = Supernatant counts; (3) = Total CPM (1) + (2); (4) = Fraction bound (1)/(3); and (5) = Platelet counts per µl x 45 µl.

The competitive binding studies using 7E3 demonstrated specific platelet (GP)IIb/IIIa receptor binding for CN with both human (Fig. 7A) and canine (Fig. 7B) platelets. The concentration of CN to inhibit 50% of 7E3 binding to human

(GP)IIb/IIIa ($IC_{50}$) is 0.4 μg/ml. The $IC_{50}$ for CN for canine (GP)IIb/IIIa is 0.24 μg/ml. These studies confirm that CN inhibits platelet aggregation by binding to (GP)IIb/IIIa.

**Example 8**

***In Vivo* Thrombolytic Efficacy of CN**

CN has been studied in a reoccluding canine model of arterial thrombosis. The protein was studied initially by systemic infusion at different dosages to determine its relative potency. This data has permitted an assessment of the systemic dose needed for effective antithrombotic (antiplatelet) activity. The effects upon physiological parameters and circulating coagulation factors have also been monitored.

The model of carotid artery thrombosis in the anesthetized canine described is a modification of one developed for the study of experimentally-induced coronary artery thrombosis [Romson, J.L. et al., Thromb. Res. 17:841 (1980)]. The experimental procedure results in the formation of a platelet rich intravascular thrombus at the site of an electrolytically-induced endothelial lesion in proximity to a distal arterial stenosis. The carotid artery is selected for the experimental model, thereby allowing one vessel to be used as a control and the other to be used after administration of the thrombolytic and antithrombotic therapy. APSAC (anisoylated plasminogen streptokinase activator complex) has been used as the thrombolytic agent successfully in this model. The carotid artery response to the electrolytic injury is similar to that observed in the canine coronary artery but has the advantage of each dog demonstrating the ability to form bilateral occlusive thrombi. The lytic-antithrombotic combination of agents may then be administered to only one of the occluded vessels; this allows for an internal control and eliminates those animals that may not form thrombi due to causes unrelated to the vessel wall injury and subsequent occlusive thrombus formation, i.e., low circulating platelet counts, enhanced spontaneous thrombolysis, presence of heart worms, etc. Parameters which are recorded include repeated measures of: phasic and mean carotid artery blood flow velocity using an ultrasonic flow probe, time to thrombotic occlusion, time to recannulization, *ex vivo* platelet aggregation, prothrombin time, thrombin time, activated partial thromboplastin time, red cell and white cell counts, hematocrit, EKG profile and body temperature, before and after administration of APSAC or APSAC plus CN to separate groups of animals.

Conditioned male mongrel dogs (8-10 kg) have been used for all *in vivo* studies. Dogs are anesthetized with sodium pentobarbital, intubated and allowed to breath room air under positive pressure respiration. Arterial blood gasses and pH determinations are made every 45 minutes and appropriate adjustments made to maintain the blood gasses and arterial pH within normal limits. Both common carotid arteries and the right internal jugular vein are exposed. A catheter is inserted into the jugular vein for blood sampling and administration of the test drug. Arterial blood pressure is monitored from the cannulated femoral artery with the use of a blood pressure transducer. A Doppler flow probe is placed on each common carotid artery proximal to both the point of insertion of the intraarterial electrode and the mechanical constrictor. The constrictor is adjusted until the pulsatile flow pattern is reduced by 50% without altering mean blood flow. Blood flow velocity in the carotid vessels is monitored continuously. Fig. 8 is a schematic representation of the instrumentation of the carotid artery.

Electrolytic injury to the intimal surface of each carotid vessel is accomplished with the use of an intravascular electrode. Each intraarterial electrode is connected to the positive pole (anode) of a dual channel stimulator. The cathode is connected to a distant subcutaneous site. The current delivered to each vessel is monitored continuously and maintained at 300 μA. The anodal electrode is positioned to have the uninsulated portion in intimate contact with the endothelial surface of the vessel. Proper positioning of the electrodes in each of the carotid arteries is confirmed by visual inspection at the end of each experiment. The anodal current is applied for a maximum period of 3 hours or is terminated 30 minutes after blood flow in the involved vessel remains stable at zero flow velocity to verify having achieved formation of a stable occlusive thrombus. The right carotid artery serves as the control vessel, whereas the left carotid artery serves as the test vessel. Vessel wall injury is induced simultaneously in each carotid artery.

APSAC (0.05 U/kg) is infused as a bolus proximal to the thrombus in the left carotid artery only. The dose of APSAC has been determined as one that will consistently lyse the locally injected carotid thrombus without producing a systemic lytic effect. Thus, lysis in the uninjected right carotid should not occur. CN is given intravenously in a 10% bolus immediately following APSAC and the remaining 90% is infused over 1 hour. CN dosages ranged from 0.155 to 0.40 mg/kg; the agent was dissolved at the appropriate dose in a volume of 20 ml of sterile saline for infusion. Reperfusion is defined as the restoration of carotid artery blood flow velocity to 20% of baseline values. Potency is defined as measurable carotid artery flow velocity. Blood pressure, heart rate, and carotid artery flow velocity are monitored for 6 hours or until rethrombosis occurs.

Blood (20 ml) was withdrawn for platelet studies from the jugular cannula into a plastic syringe containing 3.2% sodium citrate as anticoagulant (1/10 citrate/blood, vol/vol). Blood was taken for platelet aggregation and whole blood cell counts at baseline 60, 120, 180, 240 and 300 minutes after the administration of CN. The platelet count was determined with a cell counter. Platelet rich plasma, the supemate present after centrifugation of anticoagulated whole blood at 140 x g for 5 minutes, was diluted with platelet poor plasma to achieve a platelet count of 200,000/$mm^3$. Platelet poor plasma was prepared after the platelet rich plasma was removed, by centrifuging the remaining blood at 12,000 x g for 10 minutes and discarding the bottom cellular layer. *Ex vivo* platelet aggregation was measured by established spectrophotometric methods with a four channel aggregometer by recording the increase in light transmission through a stirred suspension of platelet rich plasma maintained at 37° C. Aggregation was induced with arachidonic acid (0.65 mM and 0.325 mM) and ADP (20 μM and 5 μM). A subaggregatory dose of adrenaline (550 nM) was used to prime the platelets before stimulation. Values are expressed as percentage of aggregation, representing the percentage of light transmission standardized to platelet rich and platelet poor plasma samples yielding 0% and 100% of light transmission, respectively.

At the conclusion of the study protocol each vessel segment is ligated, proximal and distal to the point of injury, and removed without disturbing the intravascular thrombus. The vessel segment is opened and the intact thrombus is lifted off and weighed.

Five animals have been studied thus far with CN plus APSAC, six with APSAC alone, and a positive control group of six dogs with APSAC plus 7E3 anti-(GP)IIb/IIIa monoclonal antibody. There were essentially no changes in mean arterial blood pressure or mean heart rate following infusion of CN. Further, the carotid artery flow velocity stayed at a high level following infusion of APSAC plus CN as compared to APSAC infusion alone. In the group of animals infused with APSAC alone, the carotid artery opened for a few minutes following infusion of the lytic agent but then reoccluded and remained closed for the duration of the study. In the positive control group, the animals were infused with APSAC (0.1 U/kg) intraarterially and this was followed by a bolus of 7E3 anti-(GP)IIb/IIIa F(ab')2 (0.8 mg/kg). In these three animals, the carotid artery remained open following infusion of the combination of APSAC and 7E3 and remained open until the conclusion of the experimental protocol. In the group of five animals infused with APSAC plus CN the results were essentially the same as with the combination of 7E3 and APSAC. However, Table 1 reveals that there was a significant advantage to the combination of APSAC plus CN in terms of the residual thrombus weight. In Table 1, CTTX = CN and RCA = right carotid artery. In the group of five animals treated with this combination of agents the residual thrombus weight per kg dog weight was 1.5, versus 2.4 in the six animals in the APSAC plus 7E3 group, and 4.1 in the APSAC alone group (six animals). Finally, in one of the dogs treated with APSAC plus CN (0.155 mg/kg) platelet aggregation and platelet counts were followed (Fig. 9); CN infusion began at time 0 and was continued for 60 minutes thereafter.

**TABLE 1**

**WEIGHT OF RESIDUAL THROMBUS IN CANINE CAROTID ARTERY THROMBOSIS MODEL**

| | APSAC Control | | APSAC | & 7E3 | APSAC | & CTTX |
|---|---|---|---|---|---|---|
| Dog# | Dog Weight (kg) | RCA Thrombus (mg) | Dog Weight (kg) | RCA Thrombus (mg) | Dog Weight (kg) | RCA Thrombus (mg) |
| 1 | 19.2 | 27.3 | 9.4 | 37.3 | 15.2 | 16.8 |
| 2 | 6.5 | 47.6 | 12.2 | 37.5 | 8.2 | 19.2 |
| 3 | 182 | 90.5 | 17 | 53.2 | 9.2 | 3.5 |
| 4 | 20.2 | 60.1 | 11.6 | 3.2 | 8.5 | 33.1 |
| 5 | 16.8 | 67.5 | 8.6 | 36.5 | 9 | 15.1 |
| 6 | 10 | 78.5 | 15.9 | 11.8 | | |
| | | | | | | |
| Mean | 15.2 | 61.9 | 12.5 | 29.9 | 10 | 14.6 |
| SEM | 2.1 | 8.4 | 1.4 | 7.6 | 1.4 | 4.9 |
| Thrombus weight per kg | | 4.1 | | 2.4 | | 1.5 |

These results are typical of those in dogs in this group. It can be seen that platelet aggregation was compromised by treatment with the venom protein, but that there appeared to be a return of aggregation at the conclusion of the experiment. Similarly, the platelet count was also depressed during the course of the experiment. It is suspected that the platelets are sequestered in some sanctuary such as the spleen and are then released following a short residence time; platelets returning into the circulation appear to be functional. There is a drop in platelet counts to 10-20% of the baseline value, with aggregability fluctuates somewhat due to the low platelet count; however, it can be seen that platelet aggregability in the residual platelets appears to be returning to normal at the conclusion of the experimental procedure.

**Example 9**

**CN effects on mammary carcinoma adhesion & invasion**

The effect of CN on binding of highly metastatic human breast cancer cells, MDA-MB-435 cell line, to ECM proteins was examined. Human fibronectin and vitronectin were immobilized in the wells of 96-well microtiter plates. Referring to Figs. 3 and 4, CN inhibited adhesion of MDA-MB-435 to both ECM proteins in a dose dependent manner $IC_{50}$ for adhesion to fibronectin is 18 nM (Fig. 10) and for vitronectin the $IC_{50}$ is 1.5 nM (Fig. 11). CN had minimal effect on the weak adhesion seen by MDA-MB-435 cells to human type I collagen, or to rat type I collagen to which the MDA cells have a relatively strong affinity. In a variation of the above experiments, CN was immobilized. It was found that CN can support binding of MDA-MB-435 cells in a dose dependent manner. Binding of MDA-MB-435 cells to immobilized CN is blocked by an RGD peptide, GRGDSP ($IC_{50}$=0.4 mM), and by EDTA ($IC_{50}$=0.8 mM). Since integrin receptors require metal ions for non covalent association of their subunits, our findings indicate that CN binds to integrin receptors on the surface of MDA-MR-435 cells via an RGD-mediated mechanism. The finding that immobilized CN can support adhesion of MDA-MB-435 cells suggests that this binding involves cell surface receptors on the tumor cells Referring to Figs. 12 and 13, varying concentrations of GRGDSP (Fig. 12) or EDTA (Fig. 13) were used to inhibit binding of human mammary carcinoma cells to immobilized CN. CN was at 0.1 μg/well. The vertical line at each data point indicates the y-axis error bar. All experiments were conducted as three sets of triplicates for each data point.

Since adhesion of MDA-MB-43 cells to immobilized CN is completely blocked by GRGDSP and by EDTA (Figs. 12 and 13), CN binds solely to integrin receptors of MDA-MB-435 cells via an RGD-dependent mechanism.

Referring to Fig. 14, we have also demonstrated the inhibitory effect of CN on the invasion of a synthetic basement membrane by the MDA-MB-435 cells using a Matrigel-coated invasion chamber. 2.5 x $10^3$ MDA-MB-435 cells treated with various concentration ofCN were allowed to migrate across the Matrigel layer for 48 hrs. Assays at each CN concentration were performed in triplicate. Cells invaded through the Matrigel filter were fixed and stained. Invaded cells were quantitated with microscope by the mean of cell numbers in three randomly selected vision field.

**Example 10**

**CN inhibits growth and metastasis of MDA-MB-435 breast cancer in nude mouse experimental model**

A spontaneous (orthotopic) metastatic model of nude mice has been established by implantation of MDA-MB-435 cells (5 x $10^5$ in 0.1 ml) in the mammary fat pads (mfp). Palpable tumors appeared by the 10th day post-implantation. Daily injections of CN into the tumor masses of each of the groups arc carried out, started at the 14th day post-implantation. By the 8th week post-implantation, tumors were removed. The animals were allowed to survive for 2 more weeks without CN administration. The animals were then sacrificed and lung metastases were carefully examined. Referring to Fig. 15, our findings indicate that local injection of CN substantially inhibited the growth rate of the tumor. The volumes of tumor masses (mean ± S.D.) of control (dark bars), low-dosage (0.5 μg/day, gray bars), and high-dosage (5μ/day, light gray bars) CN-treated group are shown. The seven clusters of bars from left to right represent the data of pre-injection (PI, · 14th day post-implantation) and the 1st through 6th week of injection. Student t-tests were employed to test the significance of differences. * and ** indicate P<0.05 and p<0.01, respectively. The mean weight of tumors treated by high-dose CN (5μg/day) is significantly lower than control group (P<0.05). Table I shows the incidence of lung metastasis based on gross examination and counting of surface nodules. Metastatic spread in the control group is much more extensive than the high dose CN group which showed >90% inhibition of metastasis. These data demonstrate the potential therapeutic role of CN in the treatment of human breast cancer.

**TABLE 2. Effect of Contortrostatin on the Incidence of Metastatic MDA-MB-435 Breast Cancer in Nude Mouse Experimental Model.**

| Groups | Metastasis Incidence | | | |
|---|---|---|---|---|
| | In situ relapse | Mean size of relapse tumor ($mm^3$) | # of nodules in lung (median) | Other Organs |
| Control | 4/5 | 66.7 ± 51.7 | $47.5^2$ | 5/5 |

(continued)

| Groups | Metastasis Incidence | | | |
|---|---|---|---|---|
| | In situ relapse | Mean size of relapse tumor ($mm^3$) | # of nodules in lung (median) | Other Organs |
| CN (5 μg/day) | 2/6 | 49.7 ± 10.5 | 4.5 | 2/6 |

[1] Organs include: Chest wall, mediastinum, diaphragm, and pleurae
[2] In 2/5 animals, lungs are directly invaded by cancer cells from pleurae and mediastinum.

**Example 11**

**CN inhibits angiogenesis induced bv MDA-MB-435 tumor In CAM**

The hypothesis that inhibitory effect on the growth of the tumors probably results at least in part from the blockage of angiogenesis by CN has been preliminarily verified by observing tho effect of CN on tumor induced angiogenesis on chick embryo chorioallantoic membrane (CAM). MDA-MB-435 tumor masses were inoculated on CAM of 10-day chick embryos. CN at various dosages were injected intravenously into CAM on day 2 post-inoculation. Tumor induced angiogenesis and inhibitory effect ofCN on angiogenesis can be easily observed in CAM after 3 days of incubation. As shown in Fig. 16, vessels are distributed in a convergent manner with the tumor mass in the center in control embryo. Chick embryo is immunodeficient, and thus allows the growth of implanted MDA-MB-435 tumor. The embryos are incubated at 37°C with humidity at 60%. CN at various dosages was injected intravenously into CAM on day 2 post-inoculation. Tumor induced angiogenesis on the 3rd day is demonstrated the photos. On the top (Fig. 16A) is the control embryo. The vessels are distributed in a convergent manner with the tumor mass in the center. In the middle (Fig. 16B) is the CAM treated with 20 μg of CN. On the bottom (Fig. 16C) is the CAM treated by 150 μg of CN. The 20 μg CN treated embryo vessels are thinner and less dense than control; tumor mass is smaller than that on control CAM. On CAM treated by 150 μg of CN the vessels are even thinner and the convergent distribution pattern disappears completely; there is a necrotic tumor mass with volume significantly smaller than control and low dose CN, presumably due to the lack of blood supply (Fig. 16).

**Example 12**

**CN has no effect on growth of MDA-MB-435 cells *In vitro***

MDA-MB-435 cells (0.3 x 106/m]) were added to each well of a 6-well cell culture plates coated with 1/100 dilution of Matrigel. Cells were then treated with CN at various concentration. Growth curves of MDA-MB-435 cells *in vitro* without CN (circles), and with CN at 100 nM (triangles), and 500 nM (diamonds) are illustrated. Six-well cell culture plates coated with Matrigel (1/100 dilute) were seeded with 3 ml of MDA-MB-435 cell suspension (0.3 x $10^6$/ml). Cell density was determined every 24 hours. Referring to Fig.17, cells in the presence of CN proliferate equally well as control cells. The result indicate that CN has no direct cytotoxicity during *in vitro* culture of MDA-MB-435 cells.

**Example 13**

**CN Is effective and well tolerated *in vivo***

It can be concluded from the chronic experiment with nude mice mentioned above that CN is not toxic. Despite its platelet aggregation inhibitory activity, no spontaneous hemorrhage is observed during the experiment. Some bleeding at the injection sites in CN treated animals, however, was noticed.

CN is a novel antimetastatic agent We hypothesize that CN blocks several critical steps (e.g. adhesion, invasion, angiogenesis) in cancer metastasis and progression. Therefore, it is more potent than other agents which block a single step.

## Claims

1. A protein consisting essentially of purified, contortrostatin precursor or biologically active variants and fragments thereof, said protein consisting of an amino acid sequence at least 95 % homologous to:

   a) amino acid numbers 191 to 410 of SEQ ID NO: 2;
   b) amino acid numbers 1 to 190 of SEQ ID NO: 2; or

c) SEQ ID NO:2.

2. A recombinant protein according to claim 1.

3. A protein according to claim 1 consisting of an amino acid sequence selected from the group consisting of:

a) amino acid numbers 191 to 410 of SEQ ID NO: 2;
b) amino acid numbers 1 to 190 of SEQ ID NO: 2; and
c) SEQ ID NO: 2.

4. A purified protein according to claim 1 comprising a constrained Arg-Gly-Asp (RGD) sequence at the tip of a flexible peptide loop of about 13 amino acid residues flanked by two Cys residues, wherein the peptide loop is an integrin antagonist which has an amino acid sequence comprising amino acid numbers 457 to 469 of SEQ ID NO:2.

5. A protein according to claim 1 wherein said biologically active variant is a metal binding proteinase, or disintegrin.

6. A preparation of contortrostatin protein encoded by the nucleotides sequence of SEQ ID NO:1.

7. A preparation of contortrostatin protein according to claim 6, said preparation being substantially free of other snake venom components.

8. A recombinant DNA molecule comprising a DNA sequence encoding contortrostatin.

9. A purified DNA molecule consisting essentially of a nucleotide sequence encoding contortrostatin, wherein said nucleotides sequence is selected from the group consisting of:

a) nucleotide numbers 1341 to 1535 of SEQ ID NO: 1;
b) nucleotide numbers 657 to 1316 of SEQ ID NO: 1;
c) nucleotide numbers 87 to 656 of SEQ ID NO: 1;
d) nucleotide numbers 87 to 1535 of SEQ ID NO: 1; and
e) SEQ ID NO:1.

10. A vector comprising a recombinant DNA molecule according to claim 9.

11. A host cell transformed with the vector of claim 10, said host cell being selected from the group consisting of animal cells, plant cells, insect cells, yeast and other fungi and bacteria.

12. A process for producing contortrostatin comprising the step of culturing the host cell of claim 11.

13. The process of claim 12, further comprising the step of recovering the contortrostatin expressed by the host cell.

14. A recombinant DNA molecule that encodes for a protein according to any of claims 1 to 5.

**Patentansprüche**

1. Protein, bestehend im wesentlichen aus gereinigtem Contortrostatin-Vorläufer oder biologisch aktiven Varianten und Fragmenten davon, wobei das Protein aus einer Aminosäuresequenz, mindestens 95% homolog zu:

a) den Aminosäurenummern 191 bis 410 von SEQ ID NO: 2;
b) den Aminosäurenummern 1 bis 190 von SEQ ID NO: 2; oder
c) SEQ ID NO: 2,

besteht.

2. Rekombinantes Protein gemäß Anspruch 1.

3. Protein gemäß Anspruch 1, bestehend aus einer Aminosäuresequenz, ausgewählt aus der Gruppe, bestehend aus:

a) den Aminosäurenummern 191 bis 410 von SEQ ID NO: 2;
b) den Aminosäurenummern 1 bis 190 von SEQ ID NO: 2; und
c) SEQ ID NO: 2.

**4.** Gereinigtes Protein gemäß Anspruch 1, umfassend eine eingeschränkte Arg-Gly-Asp-(RGD)-Sequenz an der Spitze einer flexiblen Peptidschleife von etwa 13 Aminosäureresten, flankiert durch zwei Cys-Reste, wobei die Peptidschleife ein Integrin-Antagonist ist, welcher eine Aminosäuresequenz, umfassend die Aminosäurenummern 457 bis 469 von SEQ ID NO: 2, aufweist.

**5.** Protein gemäß Anspruch 1, wobei die biologisch aktive Variante eine Metall bindende Proteinase oder Disintegrin ist.

**6.** Zubereitung von Contortrostatin-Protein, codiert durch die Nucleotidsequenz von SEQ ID NO:1.

**7.** Zubereitung von Contortrostatin-Protein gemäß Anspruch 6, wobei die Zubereitung im wesentlichen frei von anderen Schlangengift-Komponenten ist.

**8.** Rekombinantes DNA-Molekül, umfassend eine DNA-Sequenz, codierend Contortrostatin.

**9.** Gereinigtes DNA-Molekül, bestehend im wesentlichen aus einer Nucleotidsequenz, codierend Contortrostatin, wobei die Nucleotidsequenz aus der Gruppe, bestehend aus:

a) den Nucleotidnummern 1341 bis 1535 von SEQ ID NO:1;
b) den Nucleotidnummem 657 bis 1316 von SEQ ID NO:1;
c) den Nucleotidnummern 87 bis 656 von SEQ ID NO:1;
d) den Nucleotidnummern 87 bis 1535 von SEQ ID NO:1; und
e) SEQ ID NO:1,

ausgewählt ist.

**10.** Vektor, umfassend ein rekombinantes DNA-Molekül gemäß Anspruch 9.

**11.** Wirtszelle, transformiert mit dem Vektor nach Anspruch 10, wobei die Wirtszelle aus der Gruppe, bestehend aus Tierzellen, Pflanzenzellen, Insektenzellen, Hefe und anderen Pilzen sowie Bakterien, ausgewählt ist.

**12.** Verfahren zum Herstellen von Contortrostatin, umfassend den Schritt, die Wirtszelle nach Anspruch 11 zu kultivieren.

**13.** Verfahren nach Anspruch 12, weiterhin umfassend den Schritt, das Contortrostatin, exprimiert durch die Wirtszelle, zu gewinnen.

**14.** Rekombinantes DNA-Molekül, das für ein Protein gemäß einem der Ansprüche 1 bis 5 codiert.

**Revendications**

**1.** Protéine consistant essentiellement en un précurseur de contortrostatine purifiée ou en des variantes et fragments biologiquement actifs du même, ladite protéine consistant en une séquence d'acides aminés homologue à 95% au moins:

a) aux acides aminés numéros 191 à 410 de la SEQ ID NO:2;
b) aux acides aminés numéros 1 à 190 de la SEQ ID NO:2; ou
c) à la SEQ ID NO:2.

**2.** Protéine recombinante selon la revendication 1.

**3.** Protéine selon la revendication 1, consistant en une séquence d'acides aminés sélectionnée parmi le groupe consistant en:

a) les acides aminés numéros 191 à 410 de la SEQ ID NO:2;

b) les acides aminés numéros 1 à 190 de la SEQ ID NO:2; et
c) la SEQ ID NO:2.

**4.** Protéine purifiée selon la revendication 1, comprenant une séquence Arg-Gly-Asp (RGD) contrainte à la pointe d'une boucle peptidique flexible d'environ 13 résidus d'acides aminés flanqués par deux résidus Cys, dans laquelle la boucle peptidique est un antagoniste de l'intégrine qui a une séquence d'acides aminés comprenant les acides aminés numéros 457 à 469 de la SEQ ID NO:2.

**5.** Protéine selon la revendication 1, dans laquelle ladite variante biologiquement active est une protéinase à liaison métal ou une désintégrine.

**6.** Préparation de protéine contortrostatine codée par la séquence de nucléotides de la SEQ ID NO:1.

**7.** Préparation de protéine contortrostatine selon la revendication 6, ladite préparation étant sensiblement dépourvue d'autres composants de venin de serpent.

**8.** Molécule d'ADN recombinant comprenant une séquence d'ADN codant la contortrostatine.

**9.** Molécule d'ADN purifié consistant essentiellement en une séquence de nucléotides codant la contortrostatine, où ladite séquence de nucléotides est sélectionnée parmi le groupe consistant en:

a) les nucléotides numéros 1341 à 1535 de la SEQ ID NO:1;
b) les nucléotides numéros 657 à 1316 de la SEQ ID NO:1;
c) les nucléotides numéros 87 à 656 de la SEQ ID NO:1;
d) les nucléotides numéros 87 à 1535 de la SEQ ID NO:1; et
e) la SEQ ID NO:1.

**10.** Vecteur comprenant une molécule d'ADN recombinant selon la revendication 9.

**11.** Cellule hôte transformée avec le vecteur de la revendication 10, ladite cellule hôte étant sélectionnée parmi le groupe consistant en des cellules animales, des cellules végétales, des cellules d'insecte, de levure et d'autres champignons et bactéries.

**12.** Procédé de production de contortrostatine comprenant l'étape consistant à cultiver la cellule hôte de la revendication 11.

**13.** Le procédé de la revendication 12, comprenant en outre l'étape consistant à recueillir la contortrostatine exprimée par la cellule hôte.

**14.** Molécule d'ADN recombinant qui code pour une protéine selon l'une quelconque des revendications 1 à 5.

# FIG. 1

**A. Comparison of disintegrin amino acid sequences:**

| | |
|---|---|
| Contortrostatin | DAPANPCCDAATCKLTTGSQCADGLCCDQCKFMKEGTVC-RARGDDL-DY-NGISAG---- |
| Applagin | EAGEECDCGSPENPCCDAATCKLRPGAQCAEGLCCDQCKFMKEGTVC-RARGDDVNDYCNGISAGCPRNPFH |
| Trigramin | EAGEDCDCGSPANPCCDAATCKLIPGAQCGEGLCCDQCSFIEEGTVCRIARGDDLDDYCNGRSAGCPRNPFH |
| Albolabrin | EAGEDCDCGSPANPCCDAATCKLLPGAQCGEGLCCDQCSFMKKGTICRRARGDDLDDYCNGISAGCPRNPLHA |
| Elegantin | EAGEECDCGSPENPCCDAATCKLRPGAQCADGLCCDQCRFKKKRTICRRARGDNPDDRCTGQSADCPRNGLYS |
| Kistrin | GKECDCSSPENPCCDAATCKLRPGAQCGEGLCCEQCKFDRAGKICRIPRGDMPDDRCTGQSADCPRYH |

**B. Design of PCR primers:**

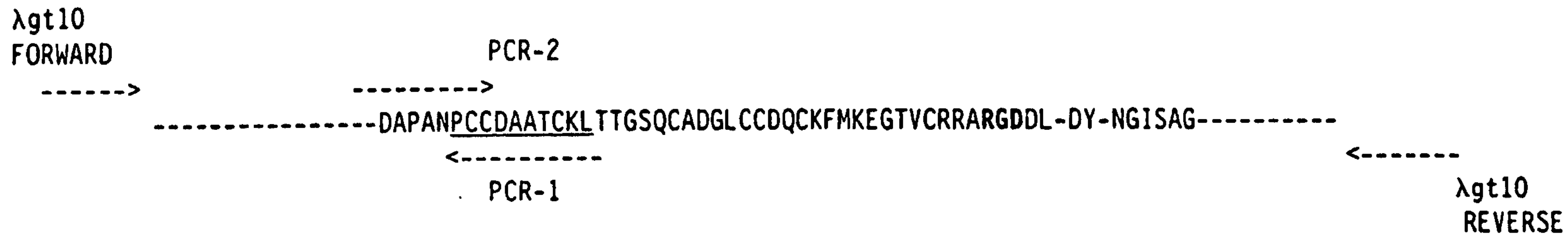


**C. Overlapping extension of PCR fragments:**

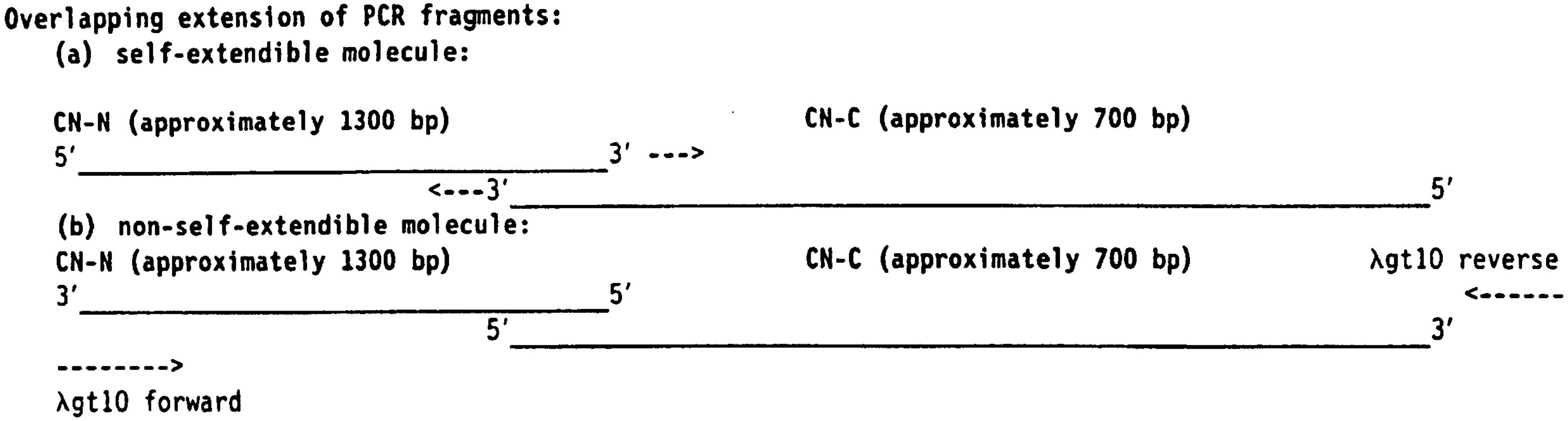

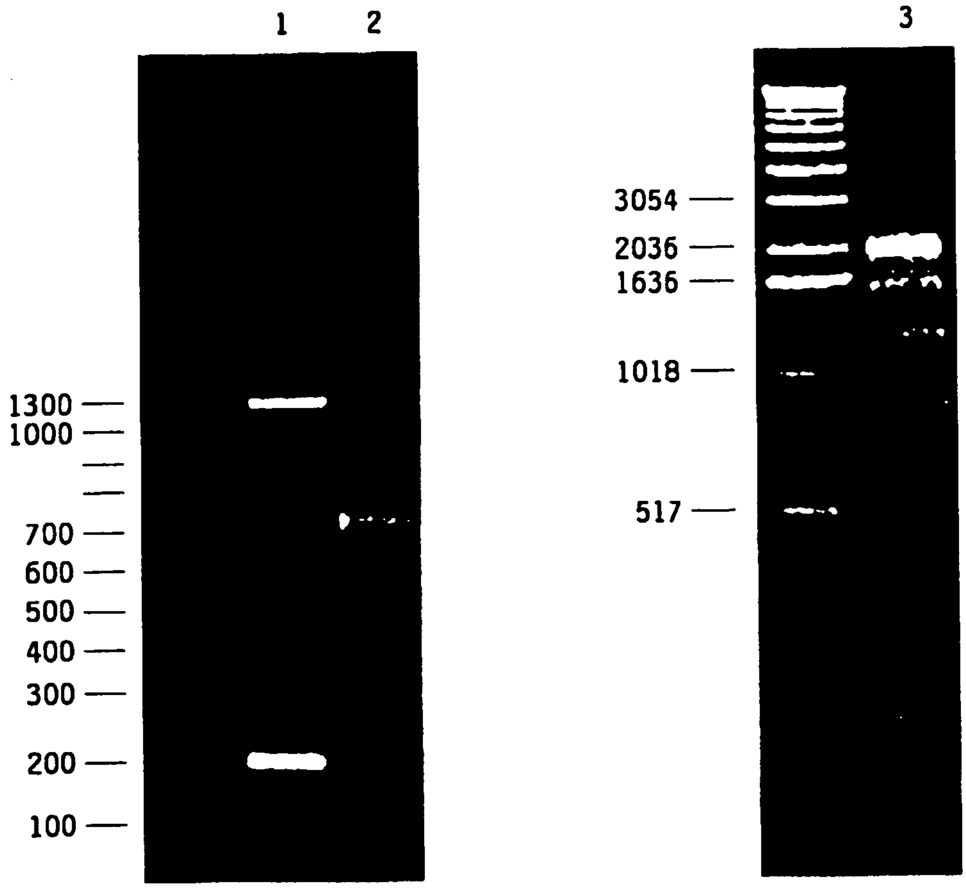
1
2
3
1300
1000
700
600
500
400
300
200
100
3054
2036
1636
1018
517

FIG. 2

# FIG. 3A

```
                    11       20       29       38       47       56
5' GA ATT CGG GGT CAA TAG AGG AAG AGC TCA AGT TGG CTT GAA AGC AGG AAG AGA TTG

            65       74       83       92      101      110
   CCT GTC TTC CAG CCA AAT CCA GCC GCC AAA ATG ATC CAG GTT CTC TTG GTG ACT
                                           --- --- --- --- --- --- --- ---
[1]                                         M   I   Q   V   L   L   V   T [8]

           119      128      137      146      155      164
   CTA TGC TTA GCA GCT TTT CCT TAT CAA GGG AGC TCT ATA ATC CTG GAA TCT GGG
   --- --- --- --- --- --- --- --- --- --- --- --- --- --- --- --- --- ---
[9] L   C   L   A   A   F   P   Y   Q   G   S   S   I   I   L   E   S   G [26]

           173      182      191      200      209      218
   AAT GTT AAT GAT TAT GAA GTA CTG TAT CCA CAA AAA GTC ACT GCA TTG CCC AAA
   --- --- --- --- --- --- --- --- --- --- --- --- --- --- --- --- --- ---
[27] N  V   N   D   Y   E   V   L   Y   P   Q   K   V   T   A   L   P   K [44]

           227      236      245      254      263      272
   GGA GCA GTT CAG CCA AAG TAT GAA GAC ACC ATG CAA TAT GAA TTT AAA GTG AAT
   --- --- --- --- --- --- --- --- --- --- --- --- --- --- --- --- --- ---
[45] G  A   V   Q   P   K   Y   E   D   T   M   Q   Y   E   F   K   V   N [62]

           281      290      299      308      317      326
   GGA GAG CCA GTG GTC CTT CAC CTG GAA AAA AAT AAA GGA CTT TTT TCA AAA GAT
   --- --- --- --- --- --- --- --- --- --- --- --- --- --- --- --- --- ---
[63] G  E   P   V   V   L   H   L   E   K   N   K   G   L   F   S   K   D [80]

           335      344      353      362      371      380
   TAC AGC GAG ACT CAT TAT TCC TCT GAT GGC AGA AAA ATT ACA ACA AAC CCT CCG
   --- --- --- --- --- --- --- --- --- --- --- --- --- --- --- --- --- ---
[81] Y  S   E   T   H   Y   S   S   D   G   R   K   I   T   T   N   P   P [98]

           389      398      407      416      425      434
   GTT GAG GAT CAC TGC TAT TAT CAT GGA CGC ATC CAG AAT GAT GCT GAC TCA ACT
   --- --- --- --- --- --- --- --- --- --- --- --- --- --- --- --- --- ---
[99] V  E   D   H   C   Y   Y   H   G   R   I   Q   N   D   A   D   S   T [116]

           443      452      461      470      479      488
   GCA AGC ATC AGT GCA TGC AAC GGT TTG AAA GGA CAT TTC AAG CTT CAA GGG GAG
   --- --- --- --- --- --- --- --- --- --- --- --- --- --- --- --- --- ---
[117]A  S   I   S   A   C   N   G   L   K   G   H   F   K   L   Q   G   E [134]

           497      506      515      524      533      542
   ACG TAC CTT ATT GAA CCC TTG AAG CTT TCC GAC AGT GAA GCC CAT GCA GTC TAC
   --- --- --- --- --- --- --- --- --- --- --- --- --- --- --- --- --- ---
[135]T  Y   L   I   E   P   L   K   L   S   D   S   E   A   H   A   V   Y [152]

           551      560      569      578      587      596
   AAA TAT GAA AAC GTA GAA AAA GAA GAT GAG GCC CCC AAA ATG TGT GGG GTA ACC
   --- --- --- --- --- --- --- --- --- --- --- --- --- --- --- --- --- ---
[153]K  Y   E   N   V   E   K   E   D   E   A   P   K   M   C   G   V   T [170]
```

# FIG. 3B

```
       605         614         623         632         641         650
CAG ACT AAT TGG GAA TCA GAT GAG CCC ATC AAA AAG GCC TCT CAG TTA AAT CTT
--- --- --- --- --- --- --- --- --- --- --- --- --- --- --- --- --- ---
[171]Q  T   N   W   E   S   D   E   P   I   K   K   A   S   Q   L   N   L [188]

        659         668         677         686         695         704
ACT CCT GAA CAA CAA GGA TTC CCC CAA AGA TAC ATT GAG CTT GTT GTA GTT GCA
--- --- --- --- --- --- --- --- --- --- --- --- --- --- --- --- --- ---
[189]T  P   E   Q   Q   G   F   P   Q   R   Y   I   E   L   V   V   V   A [206]

        713         722         731         740         749         758
GAT CAC AGA ATG TTC ACG AAA TAC AAC GGC AAT TTA AAT ACT ATT AGA ATA TGG
--- --- --- --- --- --- --- --- --- --- --- --- --- --- --- --- --- ---
[207]D  H   R   M   F   T   K   Y   N   G   N   L   N   T   I   R   I   W [224]

        767         776         785         794         803         812
GTA CAT GAA CTT GTC AAC ACT ATG AAT GTG TTT TAC AGA CCT TTG AAT ATT CGT
--- --- --- --- --- --- --- --- --- --- --- --- --- --- --- --- --- ---
[225]V  H   E   L   V   N   T   M   N   V   F   Y   R   P   L   N   I   R [242]

        821         830         839         848         857         866
GTC TCA CTG ACT GAC CTA GAA GTT TGG TCA GAC CAA GAT TTG ATC AAC GTG CAG
--- --- --- --- --- --- --- --- --- --- --- --- --- --- --- --- --- ---
[243]V  S   L   T   D   L   E   V   W   S   D   Q   D   L   I   N   V   Q [260]

        875         884         893         902         911         920
CCA GCA GCG GCT GAT ACT TTG GAA GCA TTT GGA GAC TGG AGA GAG ACA GTC TTG
--- --- --- --- --- --- --- --- --- --- --- --- --- --- --- --- --- ---
[261]P  A   A   A   D   T   L   E   A   F   G   D   W   R   E   T   V   L [278]

        929         938         947         956         965         974
CTG AAT CGC ATA AGT CAT GAT AAT GCT CAG TTA CTC ACG GCC ATT GAG CTT GAT
--- --- --- --- --- --- --- --- --- --- --- --- --- --- --- --- --- ---
[279]L  N   R   I   S   H   D   N   A   Q   L   L   T   A   I   E   L   D [296]

        983         992        1001        1010        1019        1028
GGA GAA ACT ATA GGA TTG GCT AAC AGG GGC ACC ATG TGC GAC CCG AAG CTT TCT
--- --- --- --- --- --- --- --- --- --- --- --- --- --- --- --- --- ---
[297]G  E   T   I   G   L   A   N   R   G   T   M   C   D   P   K   L   S [314]

       1037        1046        1055        1064        1073        1082
ACA GGA ATT GTT CAG GAT CAT AGT GCA ATA AAT CTT TGG GTT GCA GTT ACA ATG
--- --- --- --- --- --- --- --- --- --- --- --- --- --- --- --- --- ---
[315]T  G   I   V   Q   D   H   S   A   I   N   L   W   V   A   V   T   M [332]

       1091        1100        1109        1118        1127        1136
GCC CAT GAG ATG GGT CAT AAT CTG GGT ATT AGT CAC GAT GGA AAT CAG TGT CAT
--- --- --- --- --- --- --- --- --- --- --- --- --- --- --- --- --- ---
[333]A  H   E   M   G   H   N   L   G   I   S   H   D   G   N   Q   C   H [350]

       1145        1154        1163        1172        1181        1190
TGC GAT GCT AAC TCA TGC ATT ATG AGT GAA GAA CTA AGA GAA CAA CTT TCC TTT
--- --- --- --- --- --- --- --- --- --- --- --- --- --- --- --- --- ---
[351]C  D   A   N   S   C   I   M   S   E   E   L   R   E   Q   L   S   F [368]
```

# FIG. 3C

```
         1199           1208           1217           1226           1235           1244
     GAG TTC AGC GAT TGT AGT CAG AAT CAA TAT CAG ACA TAT CTT ACT GAT CAT AAC
     --- --- --- --- --- --- --- --- --- --- --- --- --- --- --- --- --- ---
[369]E   F   S   D   C   S   Q   N   Q   Y   Q   T   Y   L   T   D   H   N [386]

         1253           1262           1271           1280           1289           1298
     CCA CAA TGC ATG CTC AAT GAA CCC TTG AGA ACA GAT ATT GTT TCA ACT CCA GTT
     --- --- --- --- --- --- --- --- --- --- --- --- --- --- --- --- --- ---
[387]P   Q   C   M   L   N   E   P   L   R   T   D   I   V   S   T   P   V [404]

         1307           1316           1325           1334           1343           1352
     TCT GGA AAT GAA CTT TTG GAG ACG GGA GAA GAA AGT GAC TTT GAC GCT CCT GCA
     --- --- --- --- --- --- --- --- --- --- --- --- --- --- --- --- --- ---
[405]S   G   N   E   L   L   E   T   G   E   E   S   D   F   D   A   P   A [422]

         1361           1370           1379           1388           1397           1406
     AAT CCG TGC TGC GAT GCT GCA ACA TGT AAA CTG ACA ACA GGG TCA CAG TGT GCA
     --- --- --- --- --- --- --- --- --- --- --- --- --- --- --- --- --- ---
[423]N   P   C   C   D   A   A   T   C   K   L   T   T   G   S   Q   C   A [440]

         1415           1424           1433           1442           1451           1460
     GAT GGA CTG TGT TGT GAC CAG TGC AAA TTT ATG AAA GAA GGA ACA GTA TGC CGG
     --- --- --- --- --- --- --- --- --- --- --- --- --- --- --- --- --- ---
[441]D   G   L   C   C   D   Q   C   K   F   M   K   E   G   T   V   C   R [458]

         1469           1478           1487           1496           1505           1514
     AGA GCA AGG GGT GAT GAC CTG GAT GAT TAC TGC AAT GGC ATA TCT GCT GGC TGT
     --- --- --- --- --- --- --- --- --- --- --- --- --- --- --- --- --- ---
[459]R   A   R   G   D   D   L   D   D   Y   C   N   G   I   S   A   G   C [476]

         1523           1532           1541           1550           1559           1568
     CCC AGA AAT CCC TTC CAT GCC TAA CCA ACA ATG GAG ATG GAA TGG TCT GCA GCA
     --- --- --- --- --- --- --- --- --- --- --- --- --- --- --- --- --- ---
[477]P   R   N   P   F   H   A   * [483]

         1577           1586           1595           1604           1613           1622
     ACA GGC AGT GTG TTG ATC TGA ATA CAG CCT AAT AAT CAA CCT CTG GCT TCT CTC

         1631           1640           1649           1658           1667           1676
     AGA TTT GAT CAT GGA GAT CCT TCT TCC AGA AGG TTT CAC TTC CCT CAA ATC CAA

         1685           1694           1703           1712           1721           1730
     AGA GAC CCA TCT GCC TGC ATC CTA CTA GTA AAT CAC CCT TAG CTT CCA GAT GGT

         1739           1748           1757           1766           1775           1784
     ATC CAA ATT CTG TAA TAT TTC TTC TCC ATA TTT AAT CTA TTT ACC TTT TGC TGT

         1793           1802           1811           1820           1829           1838
     AAC AAA ACC TTT TTC CTG TCA CAA AGC TCC ATG GGC ATG TAC AGC TTA TCT GCT

         1847           1856           1865           1874           1883           1892
     GTC AAG AAA AAA AAT GGC CAT TTT ACC GTT TGC CAG TTA CAA AGC ACA TTT AAT

         1901           1910           1919           1928           1937           1946
     GCA ACA AGT TCT TCC TTT TGA GCT GAT GTA TTC AAA GTC AAT GCT TCC TCT CCC
```

FIG. 3D

```
      1955        1964        1973        1982        1991        2000
AAA ATT TCA TGC TGG CTT CCC AAG ATG TAG CTG CTT CCG TCA ATA AAC AAA CTA

      2009        2018        2027
TTC TCA TTC AAA AAA AAA AAC CCG AAT TC 3'
```

# FIG. 4-1

```
Proprotein domain:
                1         10        20        30        40        50
                *         *         *         *         *         *
CN          MIQVLLVTLCLAAFPYQGSSIILESGNVNDYEVLYPQKVTALPKGAVQPKY
Trigramin   MIQVLLITICLAVFPYQGSSIILESGNLNDYEVVYPEKVTALPKGAVQQKY
Cat         MIQVLLVTICLAAFPYQGSSIILESGNVNDYEVIYPRKVTALPKGAVQPKY
Jararhagin                                          ATRPKGAVQPKY
Ht-e        MIQVLLVTICLAAFPYQGSSIILESGNVNDYEVIYPRKVTALPKGAVQPKY
                        110       120       130       140       150
                        *         *         *         *         *
CN          DHCYYHGRIQNDADSTASISACNGLKGHFKLQGETYLIEPLKLSDSEAHAV
Trigramin   DHCYYHGRIENDADSTASISACDGLKGHFKLQGEMYLIEPLELSDSEAHAV
Cat         DHCYYHGRIENDADSTASISACNGLKGHFKLQGEMYLIEPLKLPDSEAHAV
Jararhagin  DHCYYHGRIENDADSTASISACNGLKGYFKLQRETYFIEPLKLPDSEAHAV
Ht-e        DHCYYHGRIENDADSTASISACNGLKGHFKLQGEMYLIEPLKLSDSEAHAV

Metalloproteinase domain:
                        200       210       220       230       240
                        *         *         *         *         *
CN          EQQGF.PQRYIELVVVADHRMFTKYNGNLNTIRIWVHELVNTMNVFYRPLN
Trigramin   EQQRF.PQRYIKLGIFVDHGMYTKYSGNSERITKRVHQMINNINMMCRALN
Cat         EHQKYNPFRFVELFLVVDKAMVTKNNGDLDKIKTRMYEIVNTVNEIYRYMY
Jararhagin  EQQRYDPYKYIEFFVVVDQGTVTKNNGDLDKIKARMYELANIVNEIFRYLY
Ht-e        EHQ.....RYVELFIVVDHGMYTKYNGDSDKIRQRVHQMVNIMKESYTYMY
           290       300       310       320       330       340
            *         *         *         *         *         *
CN          LTAIELDGETIGLANRGTMCDPKLSTGIVQDHSAINLWVAVTMAHEMGHNL
Trigramin   LTATIFNGNVIGRAPVGGMCDPKRSVAIVRDHNAIVFVVAVTMTHEMGHNL
Cat         LTAIDL.DRVIGLAYVGSMCHPKRSTGIIQDYSEINLVVAVIMAHEMGHNL
Jarahagin   LTAIDFNGPTIGYAYIGSMCHPKRSVGIVQDYSPINLVVAVIMAHEMGHNL
Ht-e        LTSIAFDEQIIGRAYIGGICDPKRSTGVVQDHSEINLRVAVTMTHELGHNL

Disintegrin domain:
                          420       430       440       450
                          *         *         *         *
CN          ETGEESDF---DAOABOCCDAATCJKTTGSQCADGKCCDQCJFNJEGTVCR
Trigramin   EAGEDCDCGSPA...NPCCDAATCKLIPGAQCGEGLCCDQCSFIEEGTVCR
Cat         EVGEECDCGTPENCQNECCDAATCKLKSGSQCGHGDCCEQCKFSKSGTECR
Jararhagin  EVGEECDCGTPENCQNECCDAATCKLKSGSQCGHGDCCEQCKFSKSGTECR
Ht-e        EAGIECDGGSLE...NPCCYATTCKMRPGSQCAEGLCCDQCRFMKKGTVCR

C-terminal domain:
                     490       500       510       520       530
                     *         *         *         *         *
Cat         NGQPCLDNYGYCYNGNCPIMYHQCYDLFGADVYEAEDSCFERNQKGNYYGY
Jararhagin  NGQPCLDNYGYCYNGNCPIMYHQCYALFGADVYEAEDSCFKDNQKGNYYGY
                     590       600
                     *         *
Cat         PGTKCADGKVCSNGHCVDVATAY*
Jararhagin  PGTKCADGKVCSNGHCVDVATAY
```

# FIG. 4-2

```
         60        70        80        90        100
          *         *         *         *         *
EDTMQYEFKVNGEPVVLHLEKNKGLFSKDYSETHYSSDGRKITTNPPVE
EDAMQYEFKVNGEPVVLHLEKNKGLFSEDYSEIHYSPDGREITAYPSVE
EDAMQYELKVNGEPVVLHLGKNKGLFSKDYSETHYSPDGREITTYPLVE
EDAMQYEFKVNGEPVVLHLEKNKGLFSKDYSEIHYSPDGREITTYPPVE
EDTMQYELKVNGEPVVLHLEKNKGLFSKDYSETHYSFDGRKITTNPSVE
       160       170       180       190
         *         *         *         *
YKYENVEKEDEAPKMCGVTQTNWESDEPIKKASQLNLTP
FKYENVEKEDEPPKMCGVTQ.NWESYESTKKASQLNVTP
YKYENVEKEDEALKMCGVTQ.NWESYEPIKKASQLVVTA
FKYENVEKEDEAPKMCGVTQ.NWKSYEPIKKASQLAFTA
FKLKNVEKEDEAPKMCGVTQ.NWESYEPIKKASDLNLNP
```

```
        250       260       270       280
         *         *         *         *
IRVSLTDLEVWSDQDLINVQPAAADTLEAFGD.WRETVLLNRISHDNAQL
IVTTLSVLEIWSEKDLITVQ.ASAPTTLTLFGAWRETVLLNRTSHDHAQL
IHVALVGLEIWSNEDKITVKPEAGYTLNA.FGEWRKTDLLTRKKHDNAQL
MHVALVGLEIWSNGDKITVKPDVDYTLNS.FAEWRKTDLLTRKKHDNAQL
IDILLAGIEIWSNGDLINVQPASPNTLNS.FGEWRETDLLKRKSHDNAQL
        350       360       370       380       390       400       410
         *         *         *         *         *         *         *
GISHDGNQCHCDANSCIMSEELREQLSFEFSDCSQNQYQTYLTDHNPQCMLNEPLRTDIVSTPVSGNELL
GMHHDEDKCNCN..TCIMSKVLSRQPSKYFSECSKDYYQTFLTNHNPQCILNAPLRTDTVSTPVSGNELL
GINHDSGYCSCGDYACIMRPEISPEPSTFFSNCSYFECWDFIMNHNPECILNEPLGTDIISPPVCGNELL
GIHHDTGSCSCGDYPCIMGPTISNEPSKFFSNCSYIQCWDFIMNHNPECIINEPLGTDIISPPVCGNELL
GIHHDTDSCSCGGYSCIMSPVISDEPSKYFSDCSYIQCWEFIMNQKPQCILKKPLRTDTVSTPVSGNELL
```

```
 460       470       480
  *         *         *
RARGD.DLDDYCNGISAGCPRNPFHA*
IARGD.DLDDYCNGRSAGCPRNPFHA
ASMSECDPAEHCTGQSSECPADVFHK
ASMSECDPAEHCTGQSSECPADVFHK
VSMVDRN.DDTCTGQSADCPRNGLYG*
```

```
    540       550       560       570       580
     *         *         *         *         *
CRKENGNKIPCAPEDVKCGRLYCKDNSPGQNNPCKMFYSNEDEHKGMVL
CRKENGKKIPCAPEDVKCGRLYCKDNSPGQNNPCKMFYSNDDEHKGMVL
```

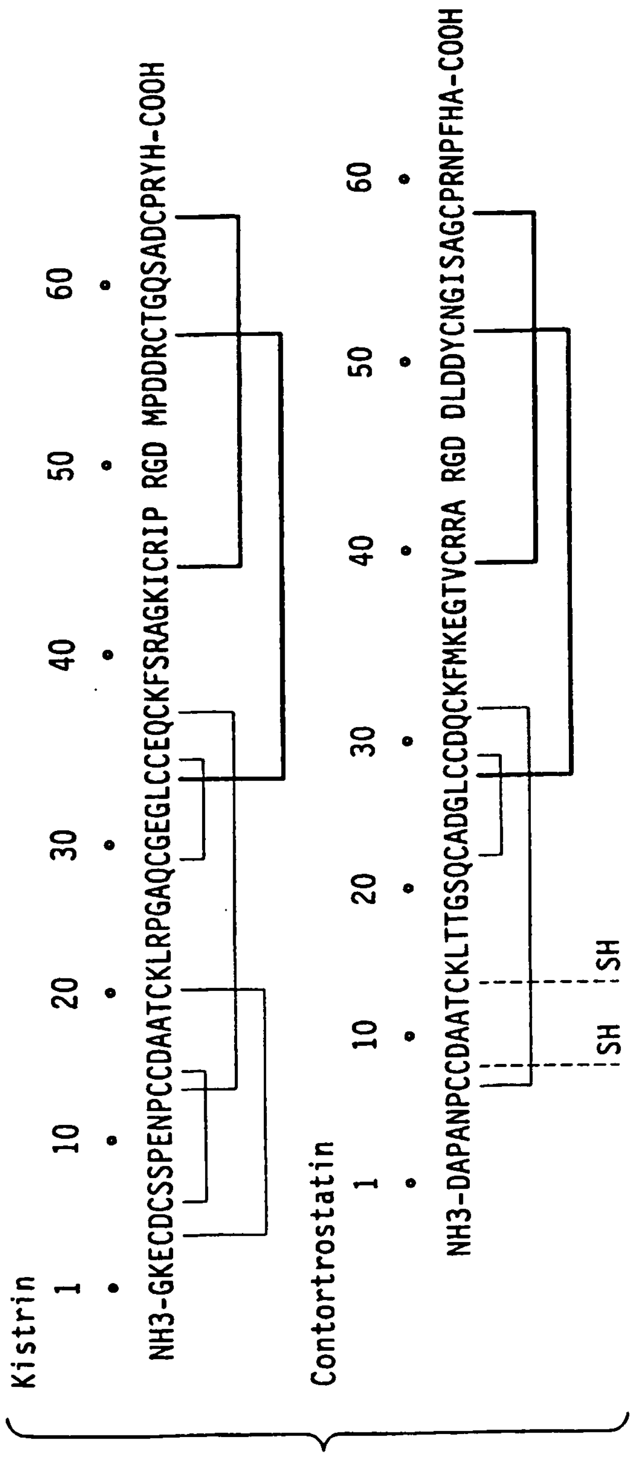
Kistrin
1 10 20 30 40 50 60
NH3-GKECDCSSPENPCCDAATCKLRPGAQCGEGLCCEQCKFSRAGKICRIP RGD MPDDRCTGQSADCPRYH-COOH
Contortrostatin
1 10 20 30 40 50 60
NH3-DAPANPCCDAATCKLTTGSQCADGLCCDQCKFMKEGTVCRRA RGD DLDDYCNGISAGCPRNPFHA-COOH
SH SH

FIG. 5A

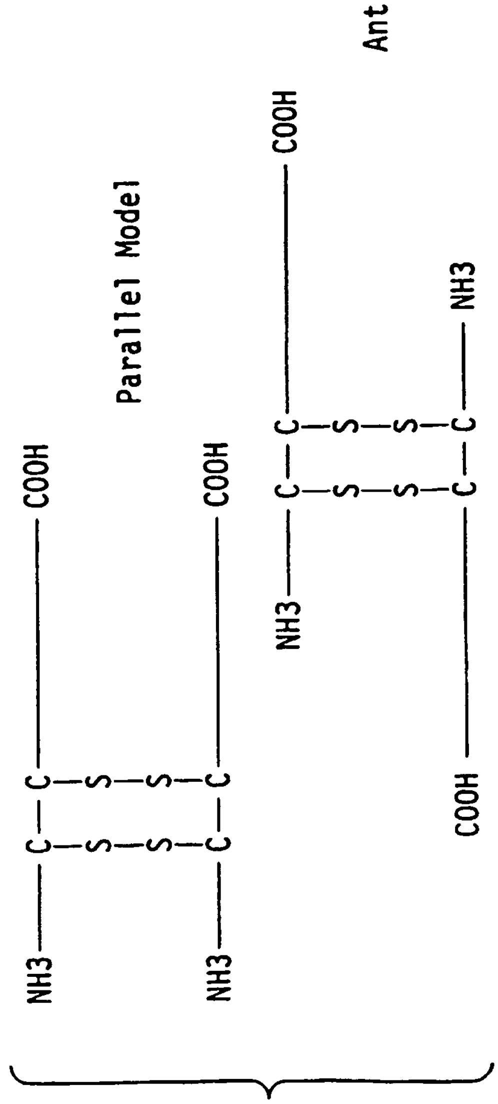
Parallel Model
NH3—C—C—COOH
S S
S S
NH3—C—C—COOH
Anti-parallel model
NH3—C—C—COOH
S S
S S
COOH—C—C—NH3

FIG. 5B

FIG. 6

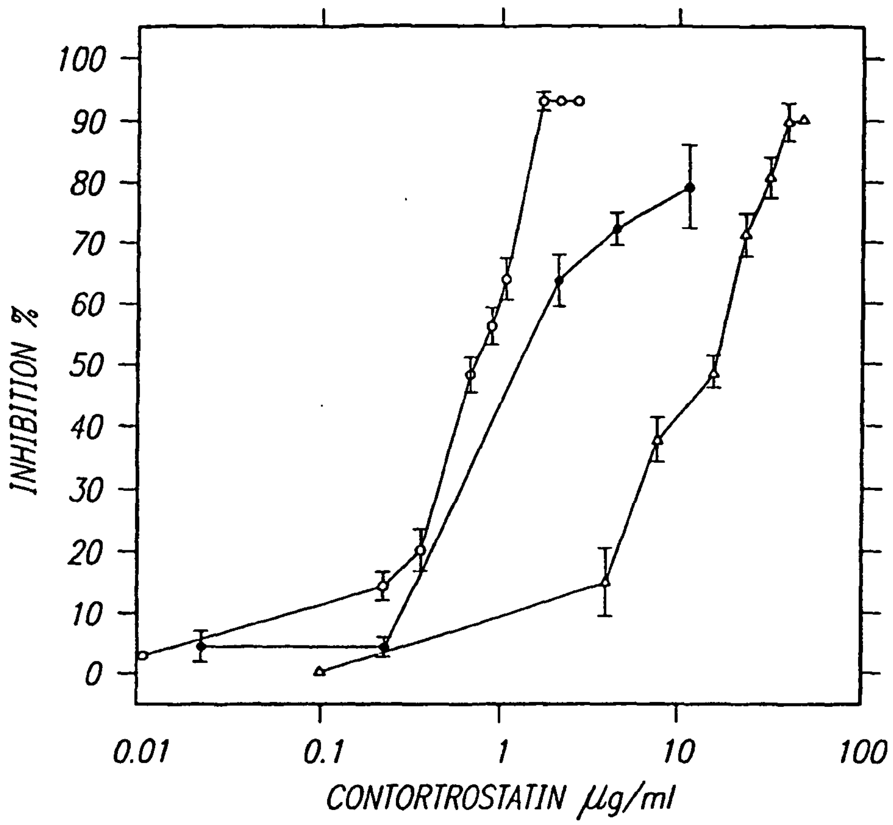
INHIBITION %
100
90
80
70
60
50
40
30
20
10
0
0.01
0.1
1
10
100
CONTORTROSTATIN μg/ml

FIG. 17

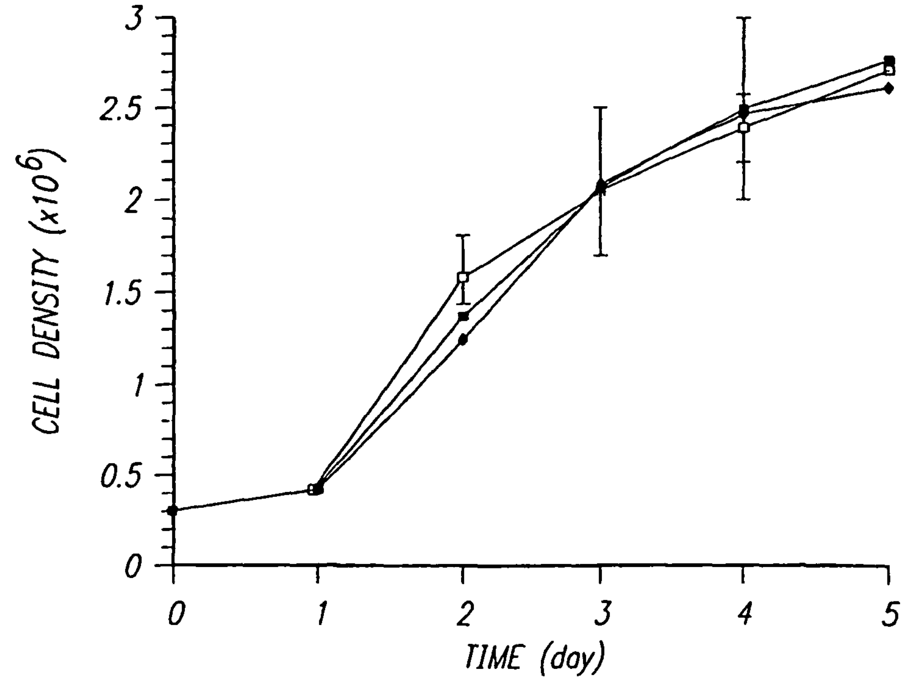
CELL DENSITY (x10^6)
3
2.5
2
1.5
1
0.5
0
0
1
2
3
4
5
TIME (day)

FIG. 7A

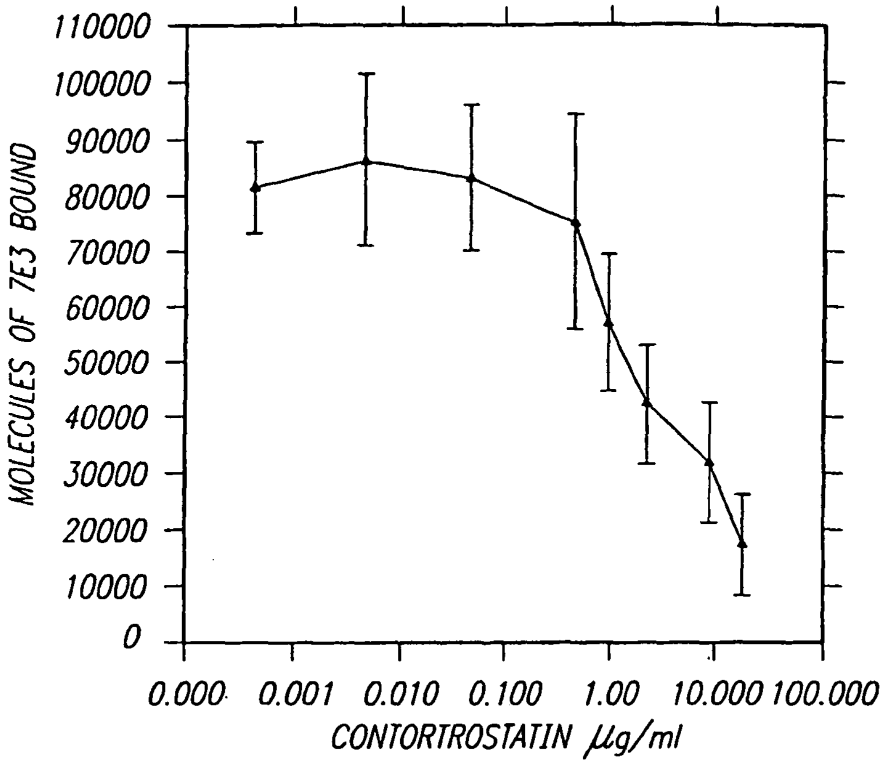
110000
100000
90000
80000
70000
60000
50000
40000
30000
20000
10000
0
MOLECULES OF 7E3 BOUND
0.000
0.001
0.010
0.100
1.00
10.000
100.000
CONTORTROSTATIN μg/ml

FIG. 7B

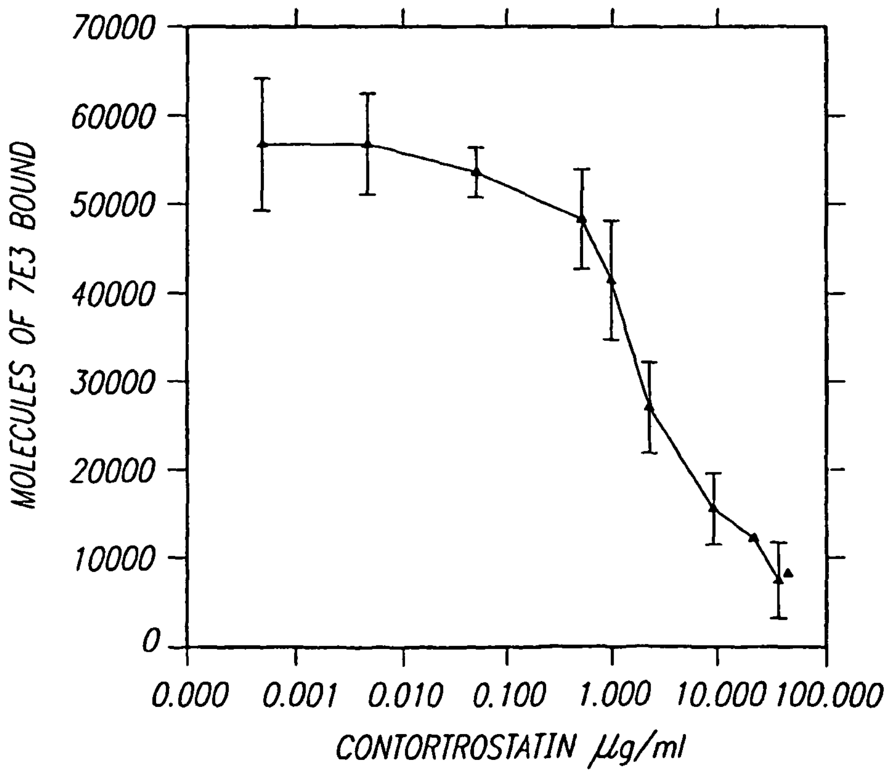
70000
60000
50000
40000
30000
20000
10000
0
MOLECULES OF 7E3 BOUND
0.000
0.001
0.010
0.100
1.000
10.000
100.000
CONTORTROSTATIN μg/ml

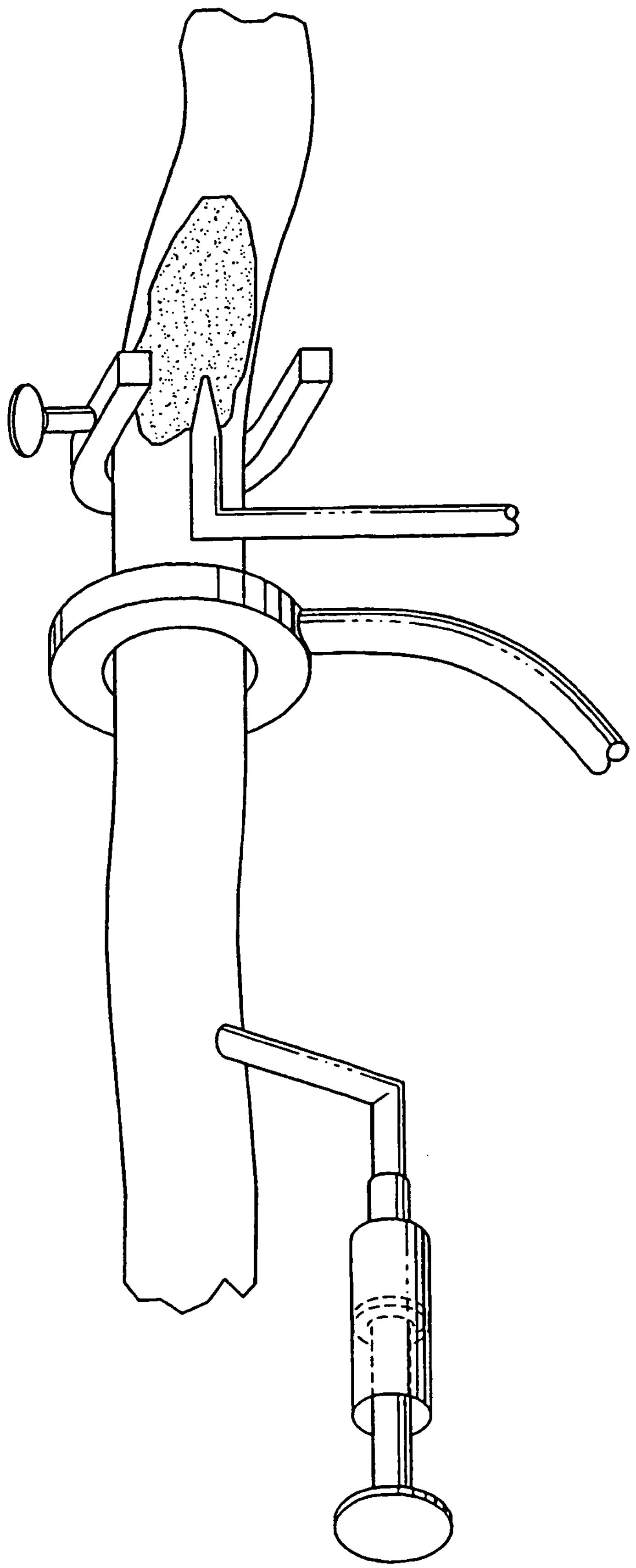

FIG. 8

FIG. 9

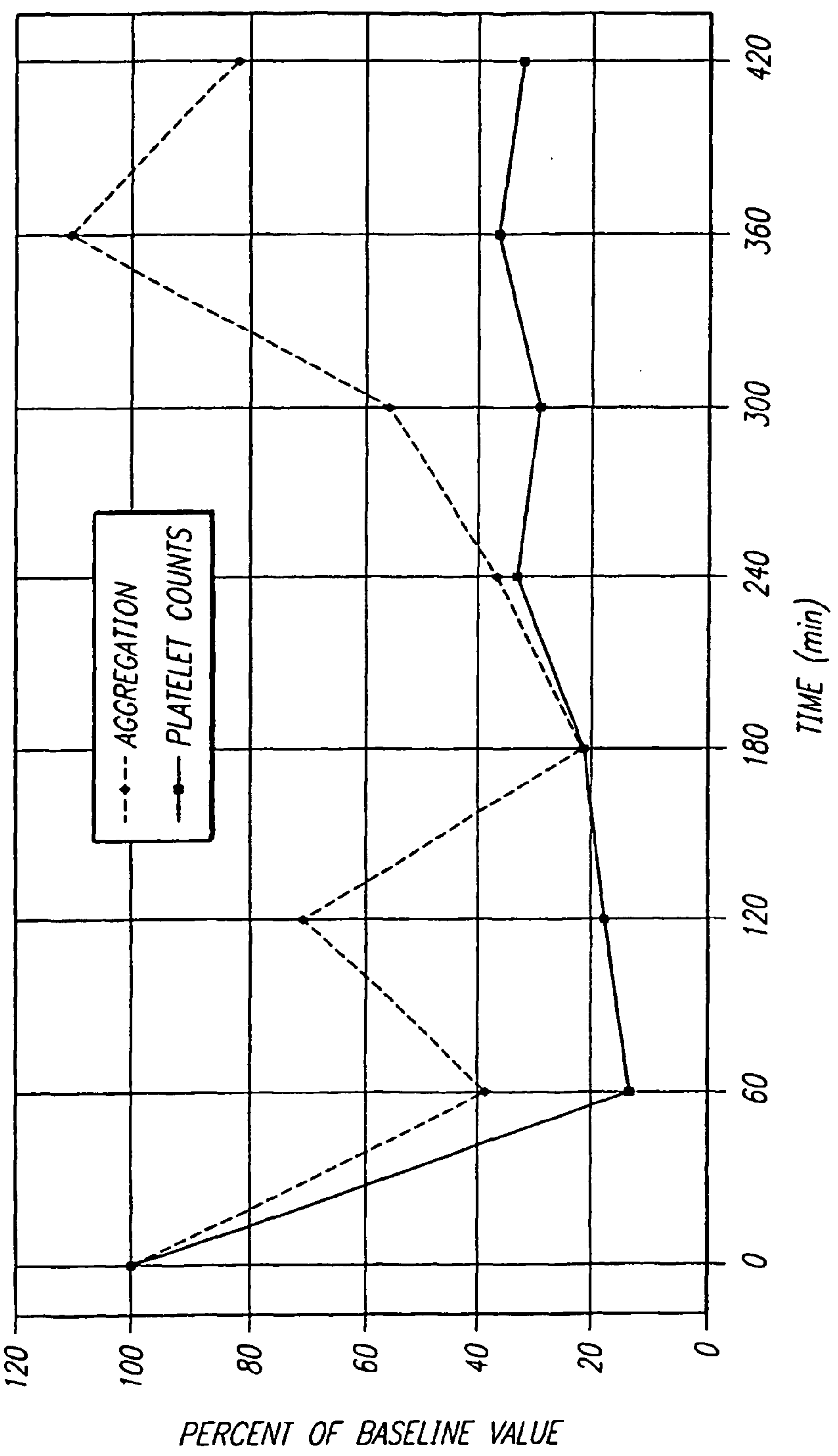
AGGREGATION
PLATELET COUNTS
TIME (min)
0
60
120
180
240
300
360
420
PERCENT OF BASELINE VALUE
120
100
80
60
40
20
0

FIG. 10

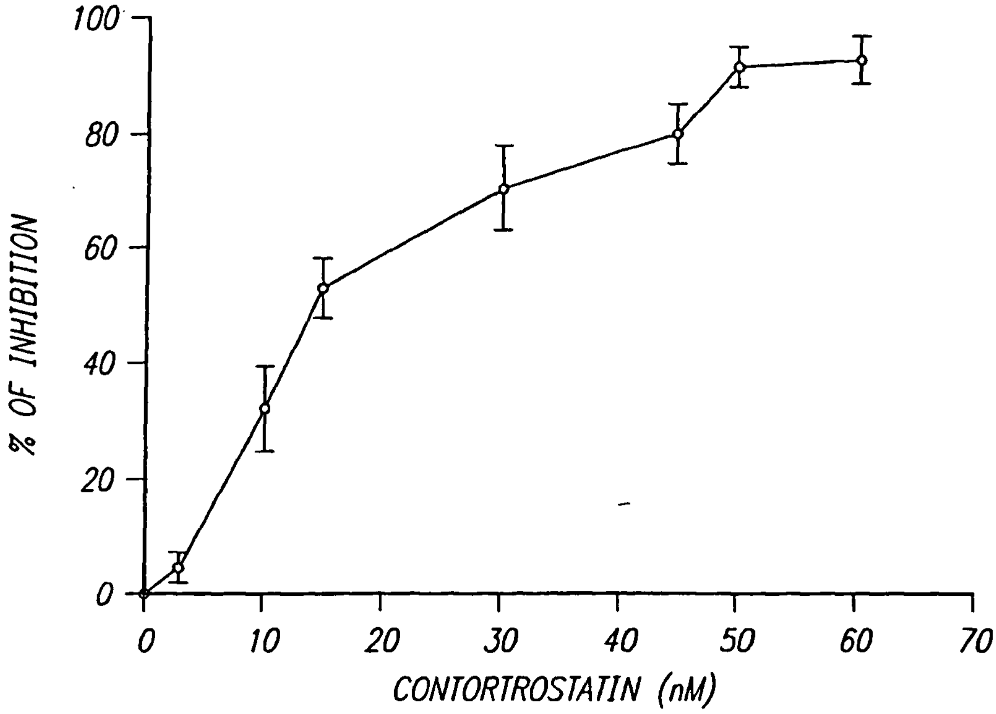
100
80
60
40
20
0
% OF INHIBITION
0
10
20
30
40
50
60
70
CONTORTROSTATIN (nM)

FIG. 11

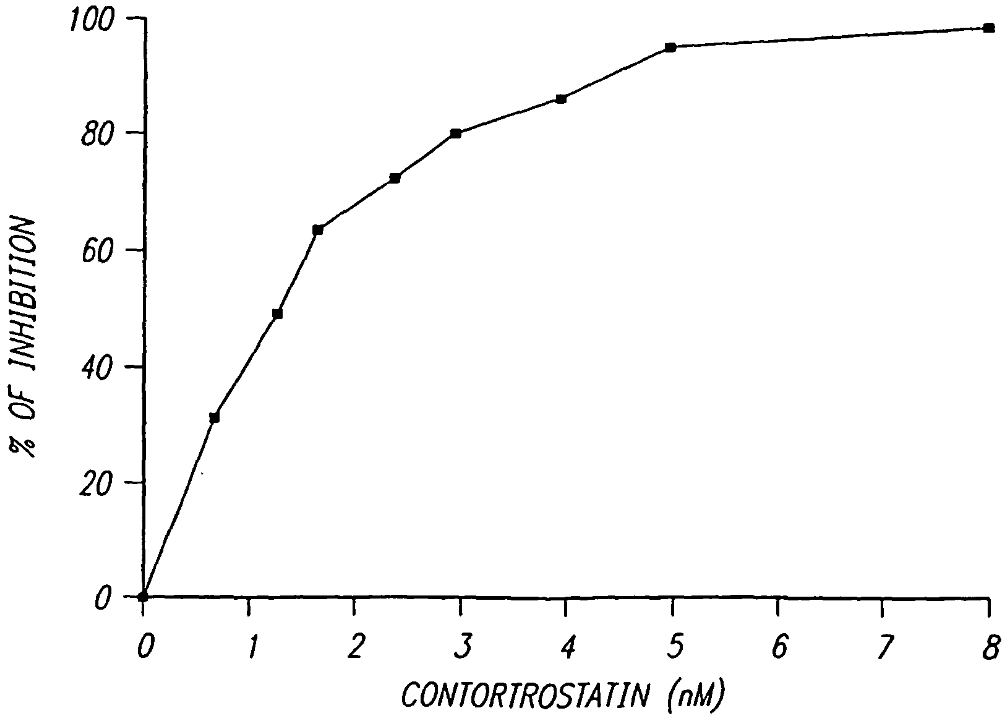
100
80
60
40
20
0
% OF INHIBITION
0
1
2
3
4
5
6
7
8
CONTORTROSTATIN (nM)

FIG. 12

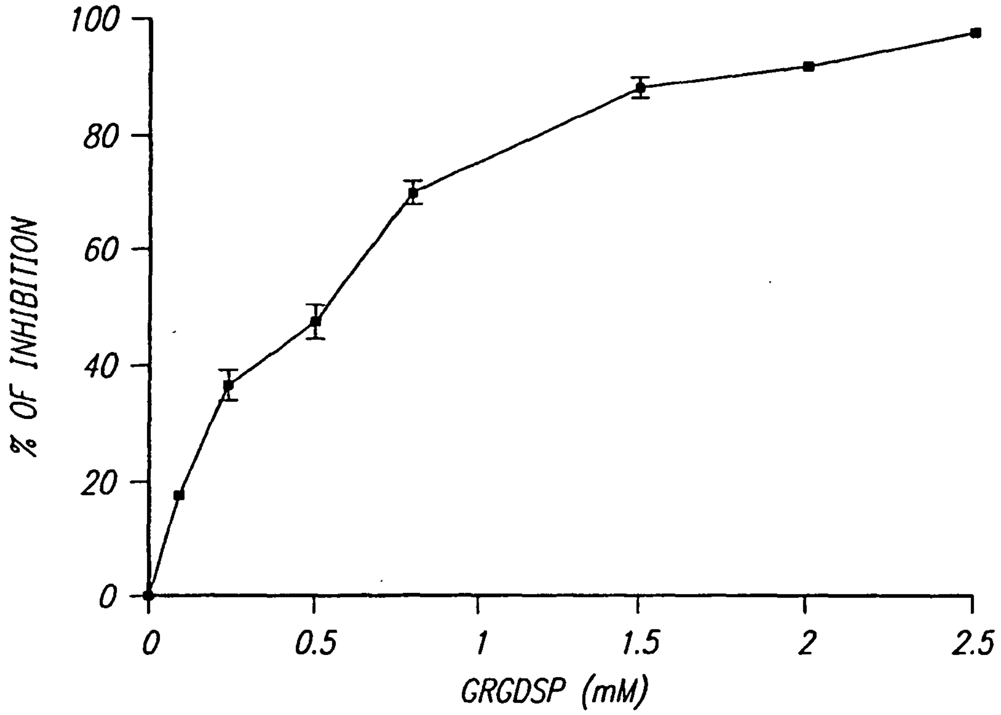
100
80
60
40
20
0
% OF INHIBITION
0
0.5
1
1.5
2
2.5
GRGDSP (mM)

FIG. 13

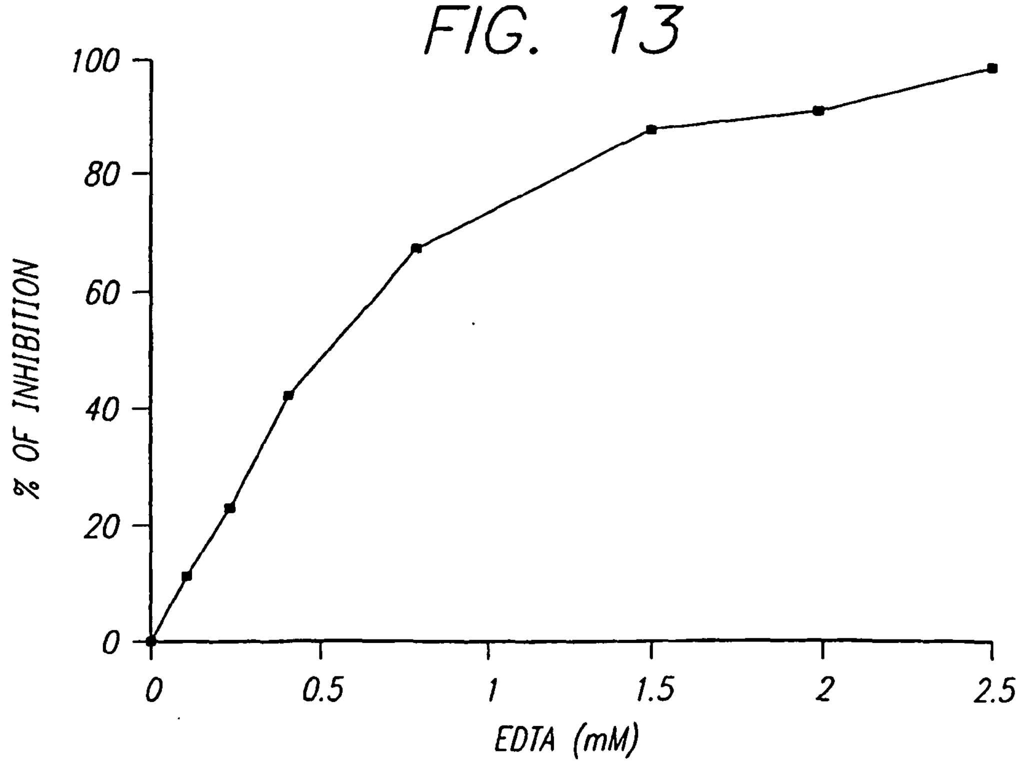
100
80
60
40
20
0
% OF INHIBITION
0
0.5
1
1.5
2
2.5
EDTA (mM)

FIG. 14

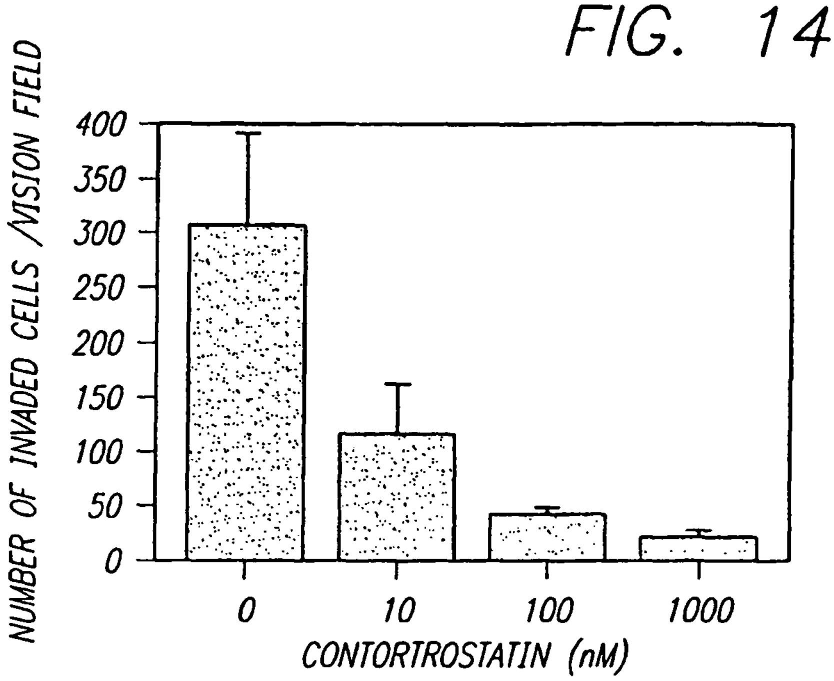
NUMBER OF INVADED CELLS /VISION FIELD
400
350
300
250
200
150
100
50
0
0
10
100
1000
CONTORTROSTATIN (nM)

FIG. 15

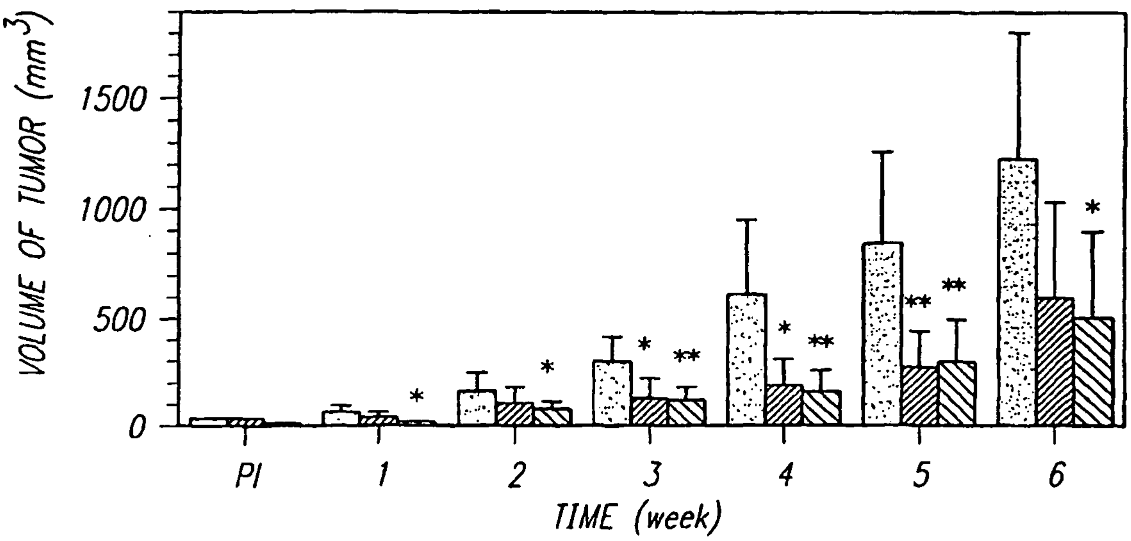
VOLUME OF TUMOR (mm3)
1500
1000
500
0
PI
1
2
3
4
5
6
TIME (week)

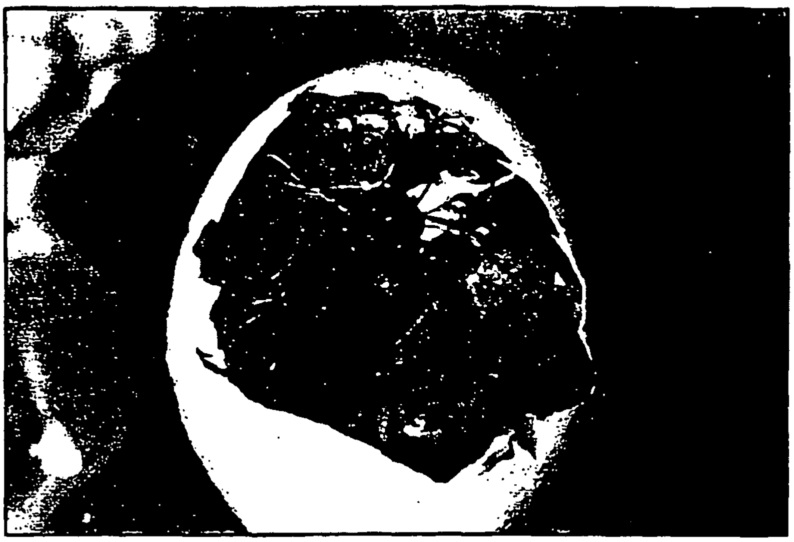

FIG. 16A

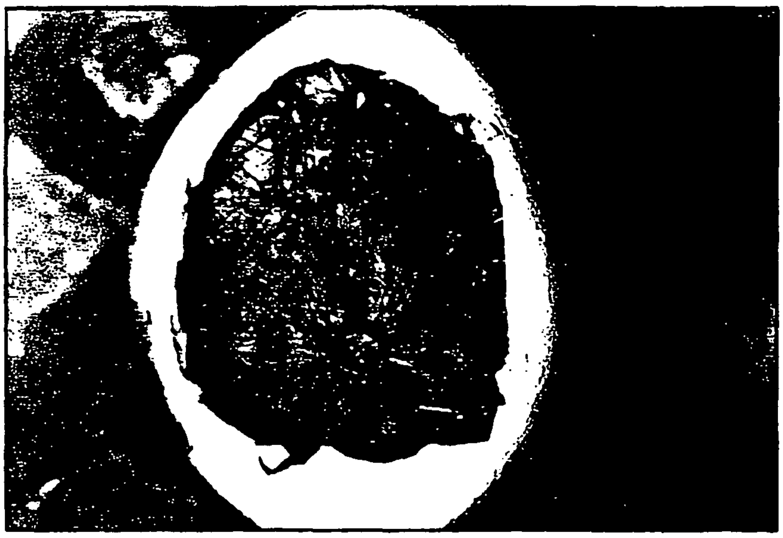

FIG. 16B

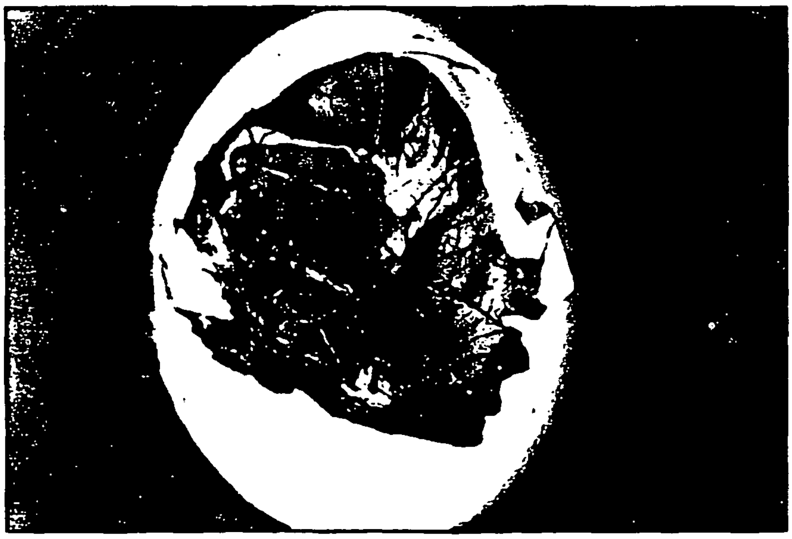

FIG. 16C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5066592 A, Huang
- US 5182260 A
- US 5196403 A, Maraganore
- US 4610879 A, Markland, Jr.
- US 09163047 B


### Non-patent literature cited in the description


- **Zucker, M.B.** *Sci. American,* 1990, vol. 242, 86
- **Ginsberg, M.H. et al.** *Thrombos. Haemostas.,* 1988, vol. 59, 1
- **Huang, T.F. et al.** *J. Biol. Chem.,* 1987, vol. 262, 16157
- **Gan, Z.R. et al.** *J. Biol Chem.,* 1988, vol. 263, 19827
- **Yasuda, T. et al.** *J. Am. Coll. Cardiol.,* 1990, vol. 16, 714
- **Trikha, M. et al.** *Fibrinolysis,* 1990, vol. 4 (1), 105
- **Trikha, M. et al.** *Blood,* 1990, vol. 76 (1), 479a
- **Holahan, M.A. et al.** *Pharmacology,* 1991, vol. 42, 340
- **Shebuski, R.J. et al.** *Circulation,* 1990, vol. 82, 169
- **Yasuda, T. et al.** *Circulation,* 1991, vol. 83, 1038
- **Scarborough, R.M. et al.** *J. Biol Chem.,* 1991, vol. 266, 9359
- **Connolly, T.M. et al.** *Circulation,* 1990, vol. 82 (III), 660
- **Chao, B.H. et al.** *Proc. Natl. Acad Sci. USA,* 1989, vol. 86, 8050
- **Savage, B. et al.** *J. Biol Chem.,* 1990, vol. 265, 11766
- **Wencel-Drake, J.D. et al.** *Blood,* 1993, vol. 81, 62
- **Trikha, M. et al.** *Journal of Cellular Biochem.,* 1992, vol. 16F, 180
- **Trikha, M. et al.** *Proceedings of the American Association for Cancer Research,* 1992, vol. 33, 34
- **Maxam ; Gilbert.** *Proc. Natl. Acad Sci. USA,* 1977, vol. 74, 560
- **Niewiarowski, S. ; McLane, M. A. ; Kloczewiak, M. ; Stewart, G. J.** Disintegrins and Other Naturally Occurring Antagonists of Platelet Fibrinogen Receptors. *Seminars in Hematology,* 1994, vol. 31, 289-300
- **Adler, M. ; Carter, P. ; Lazarus, R. A. ; Wagner, G.** Cysteine pairing in the glycoprotein IIb/IIIa antagonist kistrin using NMR, chemical analysis, and structure calculations. *Biochemistry,* 1993, vol. 32, 282-9
- **Dennis, M.S. et al.** *Proc. Natl. Acad Sci. (USA),* 1990, vol. 87, 2471
- **Coller, B.S. et al.** *J. Clin. Invest.,* 1983, vol. 72, 325
- **Sato, M. et al.** *J. Cell Biol.,* 1990, vol. 111, 1713
- **Grinnell, F.** *J. Cell. Biochem.,* 1984, vol. 26, 107
- **Clark, R.A.F.** *Arch. Dermatol.,* 1988, vol. 124, 201
- **Sible, J.C. ; Oliver, N.** *J. Cell. Biochem.,* 1992, vol. 16F, 170
- **diZerega, G.S.** *Prog. Clin. Biol. Res.,* 1993, vol. 381, 1
- **Rodgers, K. et al.** *Int. J. Fertil.,* 1990, vol. 35, 40
- **Smith, P.K. et al.** *Anal. Biochem.,* 1985, vol. 150, 76
- **Schagger, H. ; Von Jagow, G.** *Anal. Biochem.,* 1987, vol. 166, 368
- **Morrisey, J.H.** *Anal. Biochem.,* 1981, vol. 117, 307
- **Kini et al.** Structural Domains in Venom Proteins: Evidence that Metalloproteinases and Nonenzymatic Platelet Aggregation Inhibitors (Disintegrins) from Snake Venoms are Derived by Proteolysis from a Common Precursor. *Toxicon,* 1992, vol. 30, 265-296
- **Romson, J.L. et al.** *Thromb. Res.,* 1980, vol. 17, 841

SEQUENCE LISTING

<110> Harkland, Francis S.
Zhou, Qing

<120> Contortrostatin (CN) and Methods for its Use In Preventing Metastasis and Other Conditions

<130> 1920-338C3

<140> N/A
<141> 1999-09-29

<150> US 09/163,047
<151> 1998-09-29

<160> 6

<170> PatentIn Ver. 2.0

<210> 1
<211> 2029
<212> DNA
<213> Agkistrodon contortrix

<400> 1

```
gaattcgggg tcaatagagg aagagctcaa gttggcttga aagcaggaag agattgcctg 60
tcttccagcc aaatccagcc gccaaaatga tccaggttct cttggtgact ctatgcttag 120
cagcttttcc ttatcaaggg agctctataa tcctggaatc tgggaatgtt aatgattatg 180
aagtactgta tccacaaaaa gtcactgcat tgcccaaagg agcagttcag ccaaagtatg 240
aagacaccat gcaatatgaa tttaaagtga atggagagcc agtggtcctt cacctggaaa 300
aaaataaagg actttttca aaagattaca gcgagactca ttattcctct gatggcagaa 360
aaattacaac aaaccctccg gttgaggatc actgctatta tcatggacgc atccagaatg 420
atgctgactc aactgcaagc atcagtgcat gcaacggttt gaaaggacat ttcaagcttc 480
aaggggagac gtaccttatt gaacccttga agctttccga cagtgaagcc catgcagtct 540
acaaatatga aaacgtagaa aaagaagatg aggcccccaa aatgtgtggg gtaacccaga 600
ctaattggga atcagatgag cccatcaaaa aggcctctca gttaaatctt actcctgaac 660
aacaaggatt cccccaaaga tacattgagc ttgttgtagt tgcagatcac agaatgttca 720
cgaaatacaa cggcaattta aatactatta gaatatgggt acatgaactt gtcaacacta 780
tgaatgtgtt ttacagacct ttgaatattc gtgtctcact gactgaccta gaagtttggt 840
cagaccaaga tttgatcaac gtgcagccag cagcggctga tactttggaa gcatttggag 900
actggagaga gacagtcttg ctgaatcgca taagtcatga taatgctcag ttactcacgg 960
ccattgagct tgatggagaa actataggat tggctaacag ggcaccatg tgcgacccga 1020
agctttctac aggaattgtt caggatcata gtgcaataaa tctttgggtt gcagttacaa 1080
tgccccatga gatgggtcat aatctgggta ttagtcacga tggaaatcag tgtcattgcg 1140
atgctaactc atgcattatg agtgaagaac taagagaaca actttccttt gagttcagcg 1200
attgtagtca gaatcaatat cagacatatc ttactgatca taacccacaa tgcatgctca 1260
atgaaccctt gagaacagat attgtttcaa ctccagtttc tggaaatgaa cttttggaga 1320
```

```
cgggagaaga aagtgacttt gacgctcctg caaatccgtg ctgcgatgct gcaacatgta 1380
aactgacaac agggtcacag tgtgcagatg gactgtgttg tgaccagtgc aaatttatga 1440
aagaaggaac agtatgccgg agagcaaggg gtgatgacct ggatgattac tgcaatggca 1500
tatctgctgg ctgtcccaga aatcccttcc atgcctaacc aacaatggag atggaatggt 1560
ctgcagcaac aggcagtgtg ttgatctgaa tacagcctaa taatcaacct ctggcttctc 1620
tcagatttga tcatggagat ccttcttcca gaaggtttca cttccctcaa atccaaagag 1680
acccatctgc ctgcatccta ctagtaaatc acccttagct tccagatggt atccaaattc 1740
tgtaatattt cttctccata tttaatctat ttacctttg ctgtaacaaa accttttcc 1800
tgtcacaaag ctccatgggc atgtacagct tatctgctgt caagaaaaaa aatggccatt 1860
ttaccgtttg ccagttacaa agcacattta atgcaacaag ttcttccttt tgagctgatg 1920
tattcaaagt caatgcttcc tctcccaaaa tttcatgctg gcttcccaag atgtagctgc 1980
ttccgtcaat aaacaaacta ttctcattca aaaaaaaaa cccgaattc             2029
```

<210> 2
<211> 483
<212> PRT
<213> Agkistrodon contortrix

<400> 2

```
Met Ile Gln Val Leu Leu Val Thr Leu Cys Leu Ala Ala Phe Pro Tyr
1               5                   10                  15

Gln Gly Ser Ser Ile Ile Leu Glu Ser Gly Asn Val Asn Asp Tyr Glu
            20                  25                  30

Val Leu Tyr Pro Gln Lys Val Thr Ala Leu Pro Lys Gly Ala Val Gln
        35                  40                  45

Pro Lys Tyr Glu Asp Thr Met Gln Tyr Glu Phe Lys Val Asn Gly Glu
    50                  55                  60

Pro Val Val Leu His Leu Glu Lys Asn Lys Gly Leu Phe Ser Lys Asp
65                  70                  75                  80

Tyr Ser Glu Thr His Tyr Ser Ser Asp Gly Arg Lys Ile Thr Thr Asn
                85                  90                  95

Pro Pro Val Glu Asp His Cys Tyr Tyr His Gly Arg Ile Gln Asn Asp
            100                 105                 110

Ala Asp Ser Thr Ala Ser Ile Ser Ala Cys Asn Gly Leu Lys Gly His
        115                 120                 125

Phe Lys Leu Gln Gly Glu Thr Tyr Leu Ile Glu Pro Leu Lys Leu Ser
    130                 135                 140

Asp Ser Glu Ala His Ala Val Tyr Lys Tyr Glu Asn Val Glu Lys Glu
145                 150                 155                 160
```

Asp Glu Ala Pro Lys Met Cys Gly Val Thr Gln Thr Asn Trp Glu Ser
165 170 175

Asp Glu Pro Ile Lys Lys Ala Ser Gln Leu Asn Leu Thr Pro Glu Gln
180 185 190

Gln Gly Phe Pro Gln Arg Tyr Ile Glu Leu Val Val Val Ala Asp His
195 200 205

Arg Met Phe Thr Lys Tyr Asn Gly Asn Leu Asn Thr Ile Arg Ile Trp
210 215 220

Val His Glu Leu Val Asn Thr Met Asn Val Phe Tyr Arg Pro Leu Asn
225 230 235 240

Ile Arg Val Ser Leu Thr Asp Leu Glu Val Trp Ser Asp Gln Asp Leu
245 250 255

Ile Asn Val Gln Pro Ala Ala Ala Asp Thr Leu Glu Ala Phe Gly Asp
260 265 270

Trp Arg Glu Thr Val Leu Leu Asn Arg Ile Ser His Asp Asn Ala Gln
275 280 285

Leu Leu Thr Ala Ile Glu Leu Asp Gly Glu Thr Ile Gly Leu Ala Asn
290 295 300

Arg Gly Thr Met Cys Asp Pro Lys Leu Ser Thr Gly Ile Val Gln Asp
305 310 315 320

His Ser Ala Ile Asn Leu Trp Val Ala Val Thr Met Ala His Glu Met
325 330 335

Gly His Asn Leu Gly Ile Ser His Asp Gly Asn Gln Cys His Cys Asp
340 345 350

Ala Asn Ser Cys Ile Met Ser Glu Glu Leu Arg Glu Gln Leu Ser Phe
355 360 365

Glu Phe Ser Asp Cys Ser Gln Asn Gln Tyr Gln Thr Tyr Leu Thr Asp
370 375 380

His Asn Pro Gln Cys Met Leu Asn Glu Pro Leu Arg Thr Asp Ile Val
385 390 395 400

Ser Thr Pro Val Ser Gly Asn Glu Leu Leu Glu Thr Gly Glu Glu Ser
405 410 415

Asp Phe Asp Ala Pro Ala Asn Pro Cys Cys Asp Ala Ala Thr Cys Lys
420 425 430

Leu Thr Thr Gly Ser Gln Cys Ala Asp Gly Leu Cys Cys Asp Gln Cys
435 440 445

Lys Phe Met Lys Glu Gly Thr Val Cys Arg Arg Ala Arg Gly Asp Asp
450 455 460

Leu Asp Asp Tyr Cys Asn Gly Ile Ser Ala Gly Cys Pro Arg Asn Pro
465 470 475 480

Phe His Ala

<210> 3
<211> 21
<212> DNA
<213> Trimeresurus gramineus

<400> 3
gtttacaggt tgcagcatcg c 21

<210> 4
<211> 21
<212> DNA
<213> Trimeresurus gramineus

<400> 4
gcgatgctgc aacctgtaaa c 21

<210> 5
<211> 21
<212> DNA
<213> Bacteriophage Lambda gt10

<400> 5
gcgatgctgc aacctgtaaa c 21

<210> 6
<211> 24
<212> DNA
<213> Bacteriophage Lambda gt10

<400> 6
cttatgagta tttcttccag ggta 24